(19) Europäisches Patentamt
European Patent Office
Office européen des brevets

(11) **EP 2 175 259 A1**

(12) **EUROPEAN PATENT APPLICATION**
published in accordance with Art. 153(4) EPC

(43) Date of publication:
**14.04.2010 Bulletin 2010/15**

(51) Int Cl.:
*G01N 21/77* (2006.01)     *G01N 35/00* (2006.01)

(21) Application number: **07745514.5**

(22) Date of filing: **19.06.2007**

(86) International application number:
**PCT/JP2007/062312**

(87) International publication number:
**WO 2008/155820 (24.12.2008 Gazette 2008/52)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **Beckman Coulter, Inc.**
**Brea, CA 92821 (US)**

(72) Inventor: **KUBOTA, Kiyotaka**
**Sunto-gun, Shizuoka 4110931 (JP)**

(74) Representative: **Müller-Boré & Partner**
**Patentanwälte**
**Grafinger Strasse 2**
**81671 München (DE)**

(54) **METHOD OF SPECIFYING ERROR AND ANALYZER**

(57)     Disclosed is an error specifying method of specifying an error of an analyzer that analyzes a specimen based on an optical measurement. The error specifying method specifies an error of an analysis process related to removal of a high-concentration reagent, based on a reference value, which is a measurement result obtained by use of a low-concentration reagent containing a predetermined low-concentration composition, and a measured value for error specification, which is a measurement result obtained by an analysis process using the high-concentration reagent containing a predetermined high-concentration composition. The method includes measuring the amount of light emitted from the low-concentration reagent and the amount of light emitted from the high-concentration reagent and evaluating the performance of the low-concentration reagent and the performance of the high-concentration reagent.

FIG.1

EP 2 175 259 A1

**Description**

TECHNICAL FIELD

The present invention relates to a technique for specifying an error of an analyzer that analyses a specimen based on an optical measurement.

BACKGROUND ART

Analyzers can perform an analysis process on a plurality of specimens at the same time and accurately and rapidly analyze a plurality of components. Thus, analyzers have been used in various test fields, such as an immune test, a biochemical test, and a blood transfusion test.

For example, an analyzer that performs the immune test includes a reaction system that causes a reaction between a specimen and a reagent in a reaction vessel, a removal system that removes an unreacted material in the reaction vessel, and a photometric system that measures the amount of light emitted from an immune complex, which is generated by the reaction between each reagent and specimen. The systems are disposed on respective turntables. The analyzer further includes a plurality of dispensing/transferring systems that dispense or transfer the specimen, the reagent, and a reaction solution to each system. The analyzer performs immune tests of various analysis contents (see Patent Document 1, for example).

[Patent Document 1] Japanese Patent Application Laid-open No. 2003-83988

DISCLOSURE OF INVENTION

PROBLEM TO BE SOLVED BY THE INVENTION

Conventionally, it is verified whether there is an error in an analyzer based on whether the result of analysis obtained by actually performing a series of analysis processes on a standard specimen having a known analysis result matches to the known analysis result. In other words, when the analysis result obtained by actually analyzing the standard specimen matches to the known analysis result, an operator of the analyzer judges that the analyzer is normally operated without any error. When the analysis result obtained by actually analyzing the standard specimen does not match to the known analysis result, the operator of the analyzer judges that there is an error in the analyzer.

In the conventional method using the standard specimen, however, even though the operator can recognize an error in the analyzer, it is difficult to accurately specify the process and system of the analyzer where the error occurs. In particular, the analyzer that performs the immune test has a complicated structure since it performs various analysis processes having different reaction times, different reagents, different systems, and different system usage timings. Therefore, it is very difficult to accurately specify an error.

Conventionally, the dispensing accuracy of a dispensing/transferring system is verified based on the result of a colorimetric measurement obtained by actually dispensing a reagent having predetermined absorbance characteristics into a reaction vessel. However, since the analyzer that performs the immune test does not include a colorimeter, the operator needs to use a spectrophotometer that is separate from the analyzer to perform colorimetry, which is troublesome, in order to verify the dispensing accuracy.

Furthermore, in the conventional method using the standard specimen, when the standard specimen itself is denatured, the denaturation of the standard specimen is sometimes overlooked. Therefore, in the conventional method using the standard specimen, when the denatured standard specimen is used, an accurate analysis result cannot be obtained and an error in the analyzer cannot be checked.

The invention has been made in view the above-mentioned problems, and an object of the invention is to provide an error specifying method capable of accurately and easily specifying an error of an analyzer, and to provide an analyzer and a reagent.

MEANS FOR SOLVING PROBLEM

To solve the problem described above and achieve the object, an error specifying method according to the invention of specifying an error of an analyzer that analyzes a specimen based on an optical measurement, the method specifying the error of an analysis process related to removal of a high-concentration reagent based on a reference value, which is a measurement result obtained by use of a low-concentration reagent containing a predetermined low-concentration composition, and a measured value for error specification, which is a measurement result obtained by an analysis process using the high-concentration reagent containing a predetermined high-concentration composition, includes a low-concentration reagent measuring step of performing an analysis process, which causes the composition to emit light, on the low-concentration reagent and measuring an amount of light emitted in order to acquire performance of the low-concentration reagent; a high-concentration reagent measuring step of performing the analysis process, which causes the composition to emit light, on the high-concentration reagent and measuring an amount of light emitted in order to acquire performance of the high-concentration reagent; and a reagent evaluating step of evaluating the performance of the low-concentration reagent and the performance of the high-concentration reagent based on the measurement result in the low-concentration reagent measuring step and the measurement result in the high-concentration reagent measuring step.

In the error specifying method according to the invention, further included are a measuring step for acquiring a reference value of acquiring the measurement result obtained by the use of the low-concentration reagent as the reference value; a measuring step for error specification of acquiring the measurement result obtained by the analysis process using the high-concentration reagent as the measured value for error specification; and an error specifying step of specifying the error of the analysis process related to the removal of the high-concentration reagent based on whether the measured value for error specification is within an allowable range of the reference value, wherein the low-concentration reagent measuring step, the high-concentration reagent measuring step, and the reagent evaluating step are performed before or after the measuring step for acquiring a reference value and the measuring step for error specification.

In the error specifying method according to the invention, the reagent evaluating step calculates a concentration ratio of the compositions in the low-concentration reagent and the high-concentration reagent based on the measurement result in the low-concentration reagent measuring step and the measurement result in the high-concentration reagent measuring step, and when the calculated concentration ratio is not within a predetermined allowable range, it is evaluated that at least one of the low-concentration reagent and the high-concentration reagent is defective.

In the error specifying method according to the invention, further included is a zero-concentration reagent measuring step of performing the analysis process, which causes the composition to emit light, on a zero-concentration reagent that does not contain the composition and measuring an amount of light emitted, wherein the reagent evaluating step includes a low-concentration reagent evaluating step of calculating the concentration of the composition in the low-concentration reagent based on a value obtained by subtracting a measurement result in the zero-concentration reagent measuring step from the measurement result in the low-concentration reagent measuring step, and evaluating that the low-concentration reagent is defective when the calculated concentration is not within a first predetermined allowable range; and a high-concentration reagent evaluating step of calculating the concentration of the composition in the high-concentration reagent based on a value obtained by subtracting the measurement result in the zero-concentration reagent measuring step from the measurement result in the high-concentration reagent measuring step, and evaluating that the high-concentration reagent is defective when the calculated concentration is not within a second predetermined allowable range.

In the error specifying method according to the invention, further included is a correction step of correcting the measurement result in the measuring step for acquiring a reference value based on the concentration ratio calculated in the reagent evaluating step, wherein the error specifying step specifies the error of the analysis process related to the removal of the high-concentration reagent using the corrected value corrected in the correction step as the reference value.

In the error specifying method according to the invention, further included are a first correction step of correcting the measurement result in the measuring step for acquiring a reference value based on the concentration of the composition in the low-concentration reagent, which is calculated in the low-concentration reagent evaluating step; and a second correction step of correcting the measurement result in the measuring step for error specification based on the concentration of the composition in the high-concentration reagent, which is calculated in the high-concentration reagent evaluating step, wherein the error specifying step uses the corrected value corrected in the first correction step as the reference value, and uses the corrected value corrected in the second correction step as the measured value for error specification to specify the error of the analysis process related to the removal of the high-concentration reagent.

In the error specifying method according to the invention, further included is a dilution rate setting step of setting a dilution rate of the high-concentration reagent based on the measured value of light emitted during manufacture of the high-concentration reagent and a range in which a photometric system of the analyzer can measure light, wherein the high-concentration reagent measuring step performs the analysis process of causing the composition to emit light on the high-concentration reagent which is diluted at the dilution rate set in the dilution rate setting step, and measures the amount of light emitted.

In the error specifying method according to the invention, the measuring step for acquiring a reference value includes a first measuring step of measuring the amount of light emitted by the analysis process, which causes the composition in the low-concentration reagent to emit light, as the reference value, the measuring step for error specification includes a removal step of performing the same process as a removal process of removing an unreacted material in a reaction vessel included in analysis processes for the specimen to remove the composition in the high-concentration reagent accommodated in the reaction vessel, after the high-concentration reagent is injected into the reaction vessel; and a second measuring step of measuring the amount of light emitted by the analysis process that causes the composition to emit light as the measured value for error specification, and in the error specifying step, when the measured value for error specification is not within the allowable range, it is specified that there is a removal error in the removal process included in the analysis processes for the specimen.

In the error specifying method according to the invention, the error specifying step calculates a carry-over value of the removal process based on a value obtained by dividing the measured value for error specification by the measurement result obtained in the high-concentration reagent measuring step.

In the error specifying method according to the invention, a first removal process and a second removal process are performed as the removal process, and the removal step is any one of a first removal step in which the same process as the first removal process is performed, a second removal step in which the same process as the second removal process is performed, and a third removal step in which the same processes as the first removal process and the second removal process are performed.

In the error specifying method according to the invention, in the measuring step for error specification, when the removal step is the first removal step and the measured value for error specification is not within the allowable range, it is specified that there is a removal error in the first removal process of the analysis process for the specimen, when the removal step is the second removal step and the measured value for error specification is not within the allowable range, it is specified that there is a removal error in the second removal process of the analysis process for the specimen, and when the removal step is the third removal step and the measured value for error specification is not within the allowable range, it is specified that there is a removal error in the first removal process and/or the second removal process of the analysis process for the specimen.

In the error specifying method according to the invention, the measuring step for acquiring a reference value includes a first measuring step of measuring the amount of light emitted by the analysis process, which causes the composition in the low-concentration reagent to emit light, as the reference value, the measuring step for error specification includes a high-concentration reagent injection step of injecting the high-concentration reagent into a reaction vessel using a liquid injection system that injects a liquid into the reaction vessel; a zero-concentration reagent injection step of injecting a zero-concentration reagent that does not contain the composition into a reaction vessel different from the reaction vessel having the high-concentration reagent injected thereinto, using the liquid injection system which is cleaned after the high-concentration reagent injection step; and a second measuring step of measuring, as the measured value for error specification, the amount of light emitted from the reaction vessel having the zero-concentration reagent injected thereinto after the analysis process of causing the composition to emit light is performed, and in the error specifying step, when the measured value for error specification is not within the allowable range, it is specified that there is an error in a cleaning process of the liquid injection system in the analysis process for the specimen.

In the error specifying method according to the invention, the error specifying step calculates a carry-over value of the removal process based on a value obtained by dividing the measured value for error specification by the measurement result obtained in the high-concentration reagent measuring step.

An analyzer according to the invention for analyzing a specimen based on an optical measurement, the analyzer specifying an error of an analysis process related to removal of a high-concentration reagent based on a reference value, which is a measurement result obtained by use of a low-concentration reagent containing a predetermined low-concentration composition, and a measured value for error specification, which is a measurement result obtained by an analysis process using the high-concentration reagent containing a predetermined high-concentration composition, includes a measuring unit that performs a low-concentration reagent measuring process of performing an analysis process, which causes the composition to emit light, on the low-concentration reagent and measuring an amount of light emitted in order to acquire performance of the low-concentration reagent and a high-concentration reagent measuring process of performing the analysis process, which causes the composition to emit light, on the high-concentration reagent and measuring an amount of light emitted in order to acquire performance of the high-concentration reagent; and a reagent evaluating unit that evaluates the performance of the low-concentration reagent and the performance of the high-concentration reagent based on the measurement result of the low-concentration reagent measuring process and the measurement result of the high-concentration reagent measuring process obtained by the measuring unit.

In the analyzer according to the invention, further included is a specifying unit that specifies the error of the analysis process related to the removal of the high-concentration reagent, wherein the measuring unit performs a measurement process for acquiring a reference value that acquires the measurement result obtained by the use of the low-concentration reagent as the reference value and a measurement process for error specification that acquires the measurement result obtained by the analysis process using the high-concentration reagent as the measured value for error specification, and the measuring unit performs the low-concentration reagent measuring process and the high-concentration reagent measuring process before or after the measurement process for acquiring a reference value and the measurement process for error specification, the reagent evaluating unit evaluates the performance of the low-concentration reagent and the performance of the high-concentration reagent before or after the measurement process for acquiring a reference value and the measurement process for error specification, and the specifying unit specifies the error of the analysis process related to the removal of the high-concentration reagent based on whether the measured value for error specification obtained by the measuring unit is within an allowable range of the reference value.

In the analyzer according to the invention, the reagent evaluating unit calculates a concentration ratio of the compositions in the low-concentration reagent and the high-concentration reagent based on the measurement result of the low-concentration reagent measuring process and the measurement result of the high-concentration reagent measuring process obtained by the measuring unit, and when the calculated concentration ratio is not within a predetermined allowable range, the reagent evaluating unit evaluates that at least one of the low-concentration reagent and the high-concentration reagent is defective.

In the analyzer according to the invention, the measuring unit performs a zero-concentration reagent measuring process, in which the analysis process of causing the composition to emit light is performed on a zero-concentration reagent that does not contain the composition and the amount of light emitted is measured, and the reagent evaluating unit performs a low-concentration reagent evaluating process that calculates the concentration of the composition in the low-concentration reagent based on a value obtained by subtracting a measurement result of the zero-concentration reagent measuring process from the measurement result of the low-concentration reagent measuring process obtained by the measuring unit and evaluates that the low-concentration reagent is defective when the calculated concentration is not within a first predetermined allowable range, and a high-concentration reagent evaluating process that calculates the concentration of the composition in the high-concentration reagent based on a value obtained by subtracting the measurement result of the zero-concentration reagent measuring step from the measurement result of the high-concentration reagent measuring step and evaluates that the high-concentration reagent is defective when the calculated concentration is not within a second predetermined allowable range.

In the analyzer according to the invention, the specifying unit corrects the measurement result of the measurement process for acquiring a reference value obtained by the measuring unit based on the concentration ratio calculated by the reagent evaluating unit, and specifies the error of the analysis process related to the removal of the high-concentration reagent using the corrected value as the reference value.

In the analyzer according to the invention, the specifying unit performs a first correction process that corrects the measurement result of the measurement process for acquiring a reference value obtained by the measuring unit based on the concentration of the composition in the low-concentration reagent which is calculated by the reagent evaluating unit, and a second correction process that corrects the measurement result of the measurement process for error specification obtained by the measuring unit based on the concentration of the composition in the high-concentration reagent which is calculated by the reagent evaluating unit, and the specifying unit uses the corrected value corrected in the first correction process as the reference value, and uses the corrected value corrected in the second correction process as the measured value for error specification to specify the error of the analysis process related to the removal of the high-concentration reagent.

In the analyzer according to the invention, the reagent evaluating unit sets a dilution rate of the high-concentration reagent based on the measured value of light emitted during manufacture of the high-concentration reagent and a range in which a photometric system of the analyzer can measure light, and the measuring unit performs the analysis process that causes the composition to emit light on the high-concentration reagent which is diluted at the dilution rate set by the reagent evaluating unit, and measures the amount of light emitted.

In the analyzer according to the invention, further included is a removal unit that performs a removal process of removing an unreacted material in a reaction vessel, the removal process included in analysis processes for the specimen, wherein the removal unit performs the same process as the removal process to remove the composition in the high-concentration reagent accommodated in the reaction vessel after the high-concentration reagent is injected into the reaction vessel, the measuring unit measures the amount of light emitted by the analysis process that causes the composition in the low-concentration reagent to emit light as the reference value and measures, as the measured value for error specification, the amount of light emitted by the analysis process that causes the composition to emit light after the composition in the high-concentration reagent is removed by the removal unit, and when the measured value for error specification is not within the allowable range, the specifying unit specifies that there is a removal error in the removal process included in the analysis processes for the specimen.

In the analyzer according to the invention, the specifying unit calculates a carry-over value of the removal process based on a value obtained by dividing the measured value for error specification by the measurement result obtained in the high-concentration reagent measuring process.

In the analyzer according to the invention, a first removal process and a second removal process are performed as the removal process, and the removal unit performs the same process as the first removal process, the same process as the second removal process, or the same processes as the first removal process and the second removal process to remove the composition in the high-concentration reagent in the reaction vessel.

In the analyzer according to the invention, when the removal unit performs the same process as the first removal process and the measured value for error specification is not within the allowable range, the specifying unit specifies that there is a removal error in the first removal process of the analysis process for the specimen, when the removal unit performs the same process as the second removal process and the measured value for error specification is not within the allowable range, the specifying unit specifies that there is a removal error in the second removal process of the analysis process for the specimen, and when the removal unit performs the same processes as the first removal process and the second removal process and the measured value for error specification is not within the allowable range, the specifying unit specifies that there is a removal error in the first removal process and/or the second removal process of the analysis process for the specimen.

In the analyzer according to the invention, further included is a liquid injection unit that injects a liquid into a reaction vessel, wherein the liquid injection unit injects the high-concentration reagent into the reaction vessel, is cleaned, and injects a zero-concentration reagent that does not contain the composition into a reaction vessel different from the reaction vessel having the high-concentration reagent injected thereinto, the measuring unit measures the amount of light emitted by the analysis process that causes the composition in the low-concentration reagent to emit light as the reference value, and measures, as the measured value for error specification, the amount of light emitted from the reaction vessel having the zero-concentration reagent injected thereinto after the analysis process of causing the composition to emit light is performed, and when the measured value for error specification is not within the allowable range, the specifying unit specifies that there is an error in a cleaning process of the liquid injection unit in the analysis process for the specimen.

In the analyzer according to the invention, the specifying unit calculates a carry-over value of the removal process based on a value obtained by dividing the measured value for error specification by the measurement result obtained in the high-concentration reagent measuring process.

EFFECT OF THE INVENTION

According to the invention, the analyzer evaluates the performances of a low-concentration reagent and a high-concentration reagent that are actually used to specify the error of the analyzer, and specifies the error of an analysis process related to the removal of the high-concentration reagent, based on a reference value, which is the measurement result obtained by the use of the low-concentration reagent, and a measured value for error specification, which is the measurement result obtained by the analysis process using the high-concentration reagent. Therefore, the error of the analyzer can be accurately and easily specified.

BRIEF DESCRIPTION OF DRAWINGS

FIG. 1 is a diagram schematically illustrating the structure of an analyzer according to a first embodiment.
FIG. 2 is a flowchart illustrating the procedure of an error specifying process of the analyzer shown in FIG. 1.
FIG. 3 is a flowchart illustrating the procedure of a reagent evaluating process shown in FIG. 2.
FIG. 4 is a diagram illustrating a general analysis operation performed on a specimen, which is an analysis target.
FIG. 5 is a diagram illustrating a low-concentration reagent measuring process shown in FIG. 3.
FIG. 6 is a diagram illustrating a high-concentration reagent measuring process shown in FIG. 3.
FIG. 7 is a diagram illustrating a measurement process for error specification shown in FIG. 2.
FIG. 8 is a diagram illustrating the procedure of a measurement process for acquiring a BF reference and a measurement process for specifying a BF error shown in FIG. 7.
FIG. 9 is a flowchart illustrating the procedure of a BF cleaning process for specification shown in FIG. 8.
FIG. 10 is a diagram illustrating the measurement process for acquiring a BF reference shown in FIG. 8.
FIG. 11 is a diagram illustrating the measurement process for specifying a BF error shown in FIG. 8.
FIG. 12 is a diagram illustrating an example of a table used in the error specifying process shown in FIG. 2.
FIG. 13 is a diagram illustrating the procedure of a measurement process for acquiring a probe reference, a process using a high-concentration reagent, and a measurement process for specifying a probe error shown in FIG. 7.
FIG. 14 is a diagram illustrating a measurement process for acquiring a reference value shown in FIG. 13.
FIG. 15 is a diagram illustrating the process using the high-concentration reagent shown in FIG. 13.
FIG. 16 is a diagram illustrating a measurement process for error specification shown in FIG. 13.
FIG. 17 is a diagram illustrating the mixture of an antigen shown in (2) of FIG. 16.
FIG. 18 is a diagram illustrating an example of a table used in the error specifying process shown in FIG. 2.
FIG. 19 is a flowchart illustrating another procedure of the error specifying process of the analyzer shown in FIG. 2.
FIG. 20 is a diagram illustrating a correction process shown in FIG. 19.
FIG. 21 is a diagram schematically illustrating the structure of an analyzer according to a second embodiment.
FIG. 22 is a flowchart illustrating the procedure of an error specifying process of the analyzer shown in FIG. 21.
FIG. 23 is a flowchart illustrating the procedure of a reagent evaluating process shown in FIG. 22.
FIG. 24 is a diagram illustrating the content of a concentration calculating process shown in FIG. 23.

FIG. 25 is a diagram schematically illustrating the structure of an analyzer according to a third embodiment.

FIG. 26 is a flowchart illustrating the procedure of an error specifying process of the analyzer shown in FIG. 25.

FIG. 27 is a diagram illustrating a correction rate in a correction process shown in FIG. 26.

FIG. 28 is a diagram illustrating another example of the correction rate in the correction process shown in FIG. 26.

FIG. 29 is a flowchart illustrating another procedure of the error specifying process of the analyzer shown in FIG. 2.

EXPLANATIONS OF LETTERS OR NUMERALS

1, 201, 301: Analyzer
2: Measurement system
4, 204, 304: Control system
21: Specimen transfer unit
21a: Specimen vessel
21b: Specimen rack
22: Chip storage unit
23: Specimen dispensing/transferring system
24: Immune reaction table
24a: Outer circumferential line
24b: Intermediate circumferential line
24c: Inner circumferential line
25: BF table
26: First reagent storage unit
27: Second reagent storage unit
28: First reagent dispensing/transferring system
29: Second reagent dispensing/transferring system
30: Enzyme reaction table
31: Photometric system
32: First cuvette transferring system
33: Second cuvette transferring system
41: Control unit
42: Process control unit
43: Input unit
44: Analyzing unit
45, 245, 345: Specifying unit
46, 246: Reagent evaluating unit
47: Storage unit
48: Output unit
49: Transmitting/receiving unit

BEST MODE(S) FOR CARRYING OUT THE INVENTION

Hereinafter, an analyzer according to an embodiment of the invention that uses magnetic particles as solid carriers to perform an immune test, such as the antigen-antibody reaction of a test blood, among the fields of biochemistry or a blood transfusion test will be described with reference to the accompanying drawings. The invention is not limited to this embodiment. In the drawings, the same components are denoted by the same reference numerals.

(First embodiment)

Now, a first embodiment of the invention will be described. In the first embodiment, a case in which the performances of a low-concentration reagent and a high-concentration reagent that are actually used to specify the error of the analyzer are evaluated and then the error of the analyzer is specified using the low-concentration reagent and the high-concentration reagent will be described.

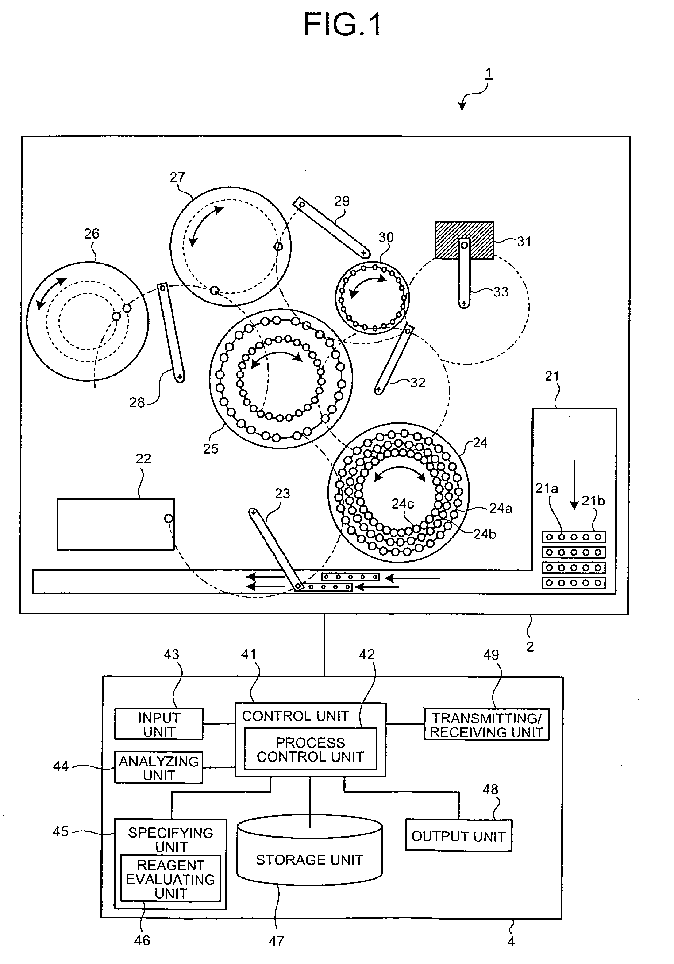

FIG. 1 is a diagram schematically illustrating the structure of the analyzer according to the first embodiment. As shown in FIG. 1, an analyzer 1 according to the first embodiment includes a measurement system 2 that measures light emitted by the reaction between a specimen and a reagent, and a control system 4 that controls the overall operation of the analyzer 1 including the measurement system 2 and analyzes the measurement results of the measurement system 2. In the analyzer 1, the two systems are operated in cooperation with each other to automatically perform immunological analysis on a plurality of specimens.

The measurement system 2 includes as main components a specimen transfer unit 21, a chip storage unit 22, a specimen dispensing/transferring system 23, an immune reaction table 24, a BF table 25, a first reagent storage unit 26, a second reagent storage unit 27, a first reagent dispensing/transferring system 28, a second reagent dispensing/ transferring system 29, an enzyme reaction table 30, a photometric system 31, a first cuvette transferring system 32, and a second cuvette transferring system 33. Each component of the measurement system 2 includes a single unit or a plurality of units that performs a predetermined operation. The control system 4 includes a control unit 41, an input unit 43, an analyzing unit 44, a specifying unit 45, a storage unit 47, an output unit 48, and a transmitting/receiving unit 49. The components of the measurement system 2 and the control system 4 are electrically connected to the control unit 41.

First, the measurement system 2 will be described. The specimen transfer unit 21 holds a plurality of specimen vessels 21a containing specimens, and includes a plurality of specimen racks 21b that is sequentially transferred in the direction of an arrow in FIG. 1. The specimen accommodated in the specimen vessel 21a is, for example, a blood or urine obtained from a specimen provider.

The chip storage unit 22 includes a chip case in which a plurality of chips is arranged, and the chip is supplied from the case. The chip is a disposable sample chip that is attached to the distal end of a nozzle of the specimen dispensing/transferring system 23 and is replaced everytime the specimen is dispensed in order to prevent a carry-over when infection items are measured.

The specimen dispensing/transferring system 23 includes an arm that has a probe for the suction and discharge of the specimen provided at the distal end thereof. The arm can move up and down in the vertical direction and can rotate about a vertical line that passes through the proximal end thereof. The specimen dispensing/transferring system 23 sucks using the probe the specimen in the specimen vessel 21a, which is moved to a predetermined position by the specimen transfer unit 21, rotates the arm to dispense the specimen to a cuvette, which is transferred to a predetermined position by the BF table 25, and transfers the specimen into the cuvette on the BF table 25 at a predetermined timing.

The immune reaction table 24 includes a reaction line that performs the reaction between the specimen in each cuvette and a predetermined reagent corresponding to an analytical item. The immune reaction table 24 can rotate about a vertical line that passes through the center of the immune reaction table 24 for each reaction line, and transfers the cuvette arranged on the immune reaction table 24 to a predetermined position at a predetermined timing. As shown in FIG. 1, the immune reaction table 24 may include three kinds of reaction lines, that is, an outer circumferential line 24a for a pre-process and pre-dilution, an intermediate circumferential line 24b for immune reaction between the specimen and a solid carrier reagent, and an inner circumferential line 24c for immune reaction between the specimen and an indicator reagent.

The BF table 25 performs a BF cleaning process for BF (bound-free) separation that sucks or discharges a predetermined cleaning solution to separate an unreacted material in the specimen or the reagent. The BF table 25 can rotate about a vertical line passing through the center of the BF table 25 for each reaction line, and transfers the cuvette arranged on the BF table 25 to a predetermined position at a predetermined timing. The BF table 25 includes a magnetic particle carrier collection system that collects magnetic particle carriers required for BF separation, BF cleaning nozzles for BF separation, and a stirring system that disperses the collected carriers. The BF cleaning process of the BF table 25 includes a first BF cleaning process and a second BF cleaning process, and the first BF cleaning process and the second BF cleaning process may be performed by different BF cleaning nozzles and different magnetic particle carrier collection systems.

The first reagent storage unit 26 can accommodate a plurality of reagent vessels each containing a first reagent to be dispensed in the cuvette arranged on the BF table 25. The second reagent storage unit 27 can accommodate a plurality of reagent vessels each containing a second reagent to be dispensed in the cuvette arranged on the BF table 25. The first reagent storage unit 26 and the second reagent storage unit 27 can be rotated in the clockwise direction or the counterclockwise direction by a driving system (not shown), and a desired reagent vessel is transferred to a reagent suction position of the first reagent dispensing/transferring system 28 or the second reagent dispensing/transferring system 29.

The first reagent dispensing/transferring system 28 includes an arm that has a probe for the suction and discharge of the first reagent provided at the distal end thereof. The arm can move up and down in the vertical direction and can rotate about a vertical line that passes through the proximal end thereof. The first reagent dispensing/transferring system 28 sucks using the probe the first reagent in the reagent vessel, which is moved to a predetermined position by the first reagent storage unit 26, and rotates the arm to dispense the reagent to the cuvette, which is transferred to a predetermined position by the BF table 25. A portion of the first reagent dispensing/transferring system 28 that comes into contact with the reagent is cleaned everytime the reagent is dispensed.

The second reagent dispensing/transferring system 29 has the same structure as the first reagent dispensing/ transferring system. The second reagent dispensing/transferring system 29 sucks using the probe the reagent in the reagent vessel, which is moved to a predetermined position by the second reagent storage unit 27, and rotates the arm to dispense the reagent to the cuvette, which is transferred to a predetermined position by the BF table 25. A portion of the second reagent dispensing/transferring system 29 that comes into contact with the reagent is cleaned everytime the reagent is dispensed.

The enzyme reaction table 30 is a reaction line for an enzyme reaction that emits light in the cuvette, to which a substrate solution is injected. The photometric system 31 measures light emitted from a reaction solution in the cuvette. For example, the photometric system 31 includes a photomultiplier tube that detects low-intensity light emitted by chemiluminescence, and measures the amount of light emitted. In addition, the photometric system 31 includes an optical filter and calculates the original emission intensity based on a measured value that is filtered by the optical filter in accordance with emission intensity.

The first cuvette transferring system 32 includes an arm that can move up and down in the vertical direction and can rotate about a vertical line that passes thorough the proximal end thereof to transfer the cuvette containing a liquid to predetermined positions on the immune reaction table 24, the BF table 25, the enzyme reaction table 30, a cuvette supply unit (not shown), and a cuvette discard unit (not shown) at predetermined timings. The second cuvette transferring system 33 includes an arm that can move up and down in the vertical direction and can rotate about a vertical line that passes thorough the proximal end thereof to transfer the cuvette containing a liquid to predetermined positions on the enzyme reaction table 30, the photometric system 31, and a cuvette discard unit (not shown) at predetermined timings.

Next, the control system 4 will be described. The control system 4 is implemented by one or a plurality of computer systems, and connected to the measurement system 2. The control system 4 controls the operation of the measurement system 2 and analyzes the measurement results of the measurement system 2 using various kinds of programs related to each process of the analyzer 1.

The control unit 41 is constructed with, for example, a CPU having a control function, and controls the process and operation of each component of the analyzer 1. The control unit 41 performs a predetermined input/output control on information input to or output from each component, and performs predetermined information processing on the input or output information. The control unit 41 reads the programs stored in the storage unit 47 from a memory and controls the operation of the analyzer 1. The control unit 41 includes a process control unit 42.

The analyzer 1 acquires the amount of light emitted from a low-concentration reagent containing a low-concentration antigen as a reference value, and acquires the amount of light emitted by an analysis process using a high-concentration reagent containing a high-concentration antigen as a measured value for error specification. Then, the analyzer 1 specifies an unreacted material removal error in a BF cleaning process or a probe cleaning error in the dispensing system. The process control unit 42 controls the operation of each component of the measurement system 2 in order to acquire the reference value and the measured value for error specification for specifying the error of the analyzer 1. The analyzer 1 evaluates the performances of the low-concentration reagent and the high-concentration reagent, which are actually used to specify the error of the analyzer 1, and then acquires the reference value and the measured value for error specification. The process control unit 42 controls the operation of each component of the measurement system 2 in order to acquire the measurement results required to evaluate the performances of the low-concentration reagent and the high-concentration reagent.

The input unit 43 includes a keyboard for inputting various kinds of information and a mouse for designating an arbitrary position on a screen of a display of the output unit 48, and acquires information required to analyze the specimen or information for instructing an analysis operation from the outside. The analyzing unit 44 analyzes the specimen based on the measurement results acquired by the measurement system 2.

The specifying unit 45 specifies whether there is an unreacted material removal error in the BF cleaning process that removes the unreacted material in the reaction vessel, which is one of analysis processes for the specimen. The specifying unit 45 uses the amount of light emitted from the low-concentration reagent containing a low concentration of antigen as the reference value, and specifies whether there is an unreacted material removal error in the BF cleaning process based on whether a measured value for error specification, which is the amount of light emitted after the BF cleaning process is performed on the high-concentration reagent containing a high concentration of antigen, is within the allowable range of the reference value. Furthermore, the specifying unit 45 uses the amount of light emitted from the low-concentration reagent as the reference value, and specifies whether there is a cleaning error in the dispensing system based on whether the amount of light emitted after the dispensing system dispenses the high-concentration reagent and performs a cleaning process and the dispensing/transferring system dispenses the zero-concentration reagent containing no antigen is within a predetermined allowable range of the reference value.

The specifying unit 45 includes a reagent evaluating unit 46. The reagent evaluating unit 46 evaluates the performances of the low-concentration reagent and the high-concentration reagent based on the measurement result of the low-concentration reagent and the measurement result of the high-concentration reagent, which are obtained by the operation of each component of the measurement system 2. The reagent evaluating unit 46 calculates the concentration ratio of the low-concentration reagent and the high-concentration reagent based on the measurement result of the low-concentration reagent and the measurement result of the high-concentration reagent. When the calculated concentration ratio is not within a predetermined allowable range, the reagent evaluating unit 46 evaluates that at least one of the low-concentration reagent and the high-concentration reagent is defective.

The storage unit 47 includes a hard disk that magnetically stores information and a memory that loads various programs related to the processes performed by the analyzer 1 from the hard disk and electrically stores the programs, and stores information including the analysis results of the specimen, for example. The storage unit 47 may include an auxiliary storage device that can read information stored in a storage medium, such as a CD-ROM, a DVD-ROM, or a PC card.

The output unit 48 includes, for example, a display, a printer, and a speaker, and outputs information related to analysis under the control of the process control unit 42. The transmitting/receiving unit 49 serves as an interface that transmits or receives information having a predetermined format through a communication network (not shown).

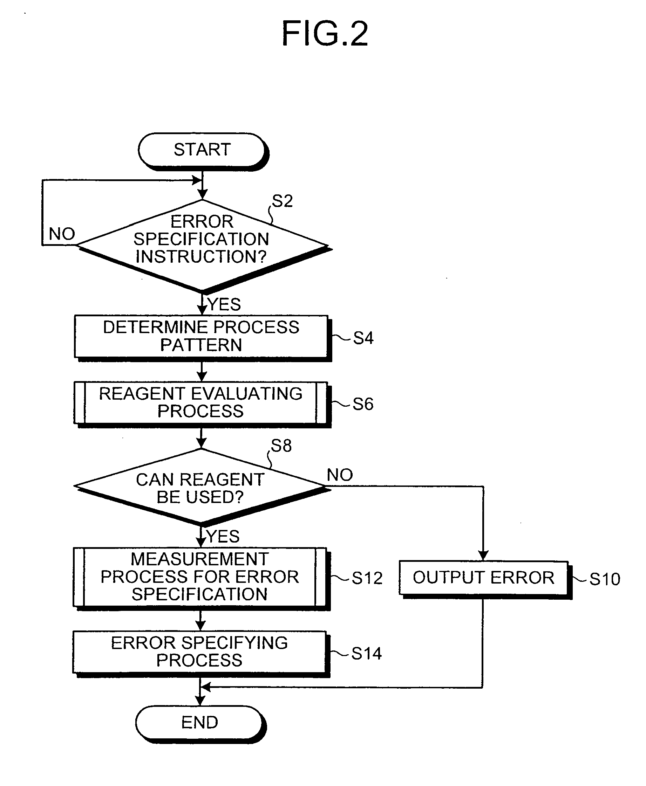

Next, the procedure of the error specifying process of the analyzer 1 will be described with reference to FIG. 2. As shown in FIG. 2, the control unit 41 determines whether the error specifying process is instructed in accordance with instruction information input from the input unit 43 that instructs the specification of the unreacted material removal error in the BF cleaning process or the specification of the cleaning error of the probe in the dispensing system (Step S2). The control unit 41 repeats a determination process of Step S2 until the error specifying process is instructed. When it is determined that the error specifying process is instructed (Step S2: Yes), the control unit 41 determines a process pattern corresponding to the cleaning process of the dispensing system or the BF cleaning process, which is a removal error specification target, in accordance with the instruction information input from the input unit 43 (Step S4). Then, the reagent evaluating unit 46 and the components of the measurement system 2 perform a reagent evaluating process that evaluate the performances of the low-concentration reagent and the high-concentration reagent that are actually used in the error specifying process and determines whether the low-concentration reagent and the high-concentration reagent can be used, under the control of the process control unit 42 (Step S6). Then, the specifying unit 45 determines whether the low-concentration reagent and the high-concentration reagent can be used based on the evaluation result of the reagent evaluating unit 46 (Step S8).

If the specifying unit 45 determines that the low-concentration reagent and the high-concentration reagent cannot be used (Step S8: No), the output unit 48 outputs an error message indicating that there is a defect in at least one of the low-concentration reagent and the high-concentration reagent and the error specifying process is not available, under the control of the control unit 41 (Step S10). The operator of the analyzer 1 confirms the error message and can know that there is a defect in at least one of the low-concentration reagent and the high-concentration reagent and it is difficult to accurately specify the error of the analyzer 1 even when the error specifying process is performed. Therefore, the operator can replace both the low-concentration reagent and the high-concentration reagent with new reagents. As such, the analyzer 1 prevents the low-concentration reagent and the high-concentration reagent whose performance has deteriorated from being used in the error specifying process.

Conversely, if the specifying unit 45 determines that the low-concentration reagent and the high-concentration reagent can be used (Step S8: Yes), each component of the measurement system 2 performs the measurement process for error specification using the low-concentration reagent and the high-concentration reagent that have been evaluated to be usable by the reagent evaluating unit 46, under the control of the process control unit 42 (Step S12). As the measurement process for error specification, the following processes are performed: a measurement process for acquiring a reference value that acquires the measurement result obtained by the use of the low-concentration reagent as a reference value; and a measurement process for specifying an error that acquires the measurement result obtained by the use of the high-concentration reagent as a measured value for error specification. The specifying unit 45 performs an error specifying process that specifies the removal error of the BF cleaning process or the cleaning error of the dispensing system, which is an error specification target, based on whether the measured value for error specification obtained in the measurement process for specifying an error is within the allowable range of the reference value obtained in the measurement process for acquiring a reference value (Step S14).

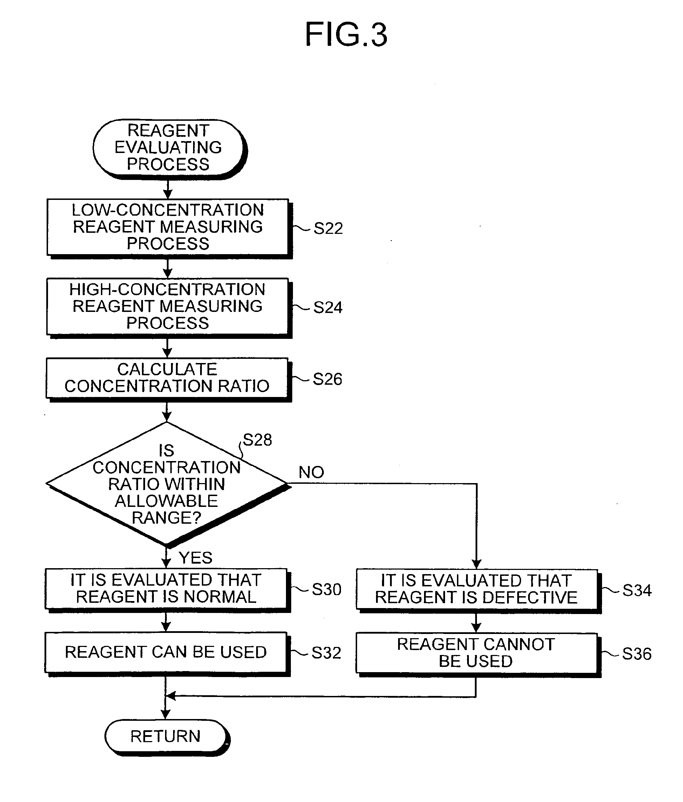

Next, the reagent evaluating process shown in FIG. 2 will be described with reference to FIG. 3. As shown in FIG. 3, the process control unit 42 controls each component of the measurement system 2 to perform a low-concentration reagent measuring process, in which an analysis process of causing an antigen to emit light, is performed on the low-concentration reagent and the amount of light emitted is measured, in order to acquire the performance of the low-concentration reagent (Step S22).

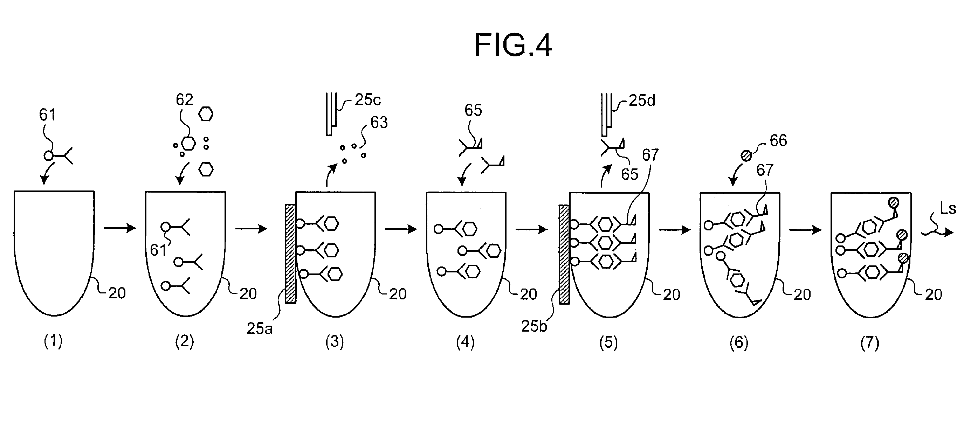

Next, the analysis process of causing the antigen to emit light will be described. First, a general analysis process that is performed in order to detect the amount of antigen 62 in the specimen will be described with reference to FIG. 4. In the analysis of the specimen, as shown in (1) of FIG. 4, a first reagent dispensing process is performed in which a cuvette 20 is transferred from a cuvette supply unit (not shown in FIG. 1) to a predetermined position on the BF table 25 by the first cuvette transferring system 32 and a first reagent containing a magnetic particle 61 is dispensed into the cuvette 20 by the first reagent dispensing/transferring system 28. Then, as shown in (2) of FIG. 4, a specimen dispensing process is performed in which the specimen dispensing/transferring system 23 having a chip supplied from the chip storage unit 22 attached thereto dispenses a specimen from the specimen vessel 21a transferred to a predetermined position by the specimen transfer unit 21 into the cuvette 20 on the BF table 25. Then, the cuvette 20 is stirred by the stirring system of the BF table 25, and is then transferred to the intermediate circumferential line 24b of the immune reaction table 24 by the first cuvette transferring system 32. In this case, after a predetermined reaction time has elapsed, the antigen 62 in the specimen is coupled to the magnetic particle 61.

Then, the cuvette 20 is transferred onto the BF table 25 by the first cuvette transferring system 32. As shown in (3) of FIG. 4, a first BF cleaning process is performed in which a magnetic particle collection system 25a of the BF table 25 collects the magnetic particles and a BF cleaning nozzle 25c performs BF separation. As a result, as shown in (3) of FIG. 4, an unreacted material 63 in the cuvette 20 is removed.

Then, as shown in (4) of FIG. 4, a second reagent dispensing process is performed in which the second reagent dispensing/transferring system 29 dispenses an indicator reagent, which is a second reagent, containing an indicator antibody 65 into the cuvette 20 as the second reagent after the BF separation and the stirring system stirs the reagent. As a result, the antigen 62, the magnetic particle 61, and the indicator antibody 65 are coupled to each other to generate an immune complex 67. Then, the cuvette 20 is transferred to the inner circumferential line 24c of the immune reaction table 24 by the first cuvette transferring system 32. Then, after a predetermined reaction time has elapsed, the cuvette 20 is transferred onto the BF table 25.

Then, as shown in (5) of FIG. 4, a second BF cleaning process is performed in which a magnetic particle collection system 25b collects the magnetic particles in the cuvette 20 and a BF cleaning nozzle 25d performs BF separation. As a result, as shown in (5) of FIG. 4, the indicator antibody 65 that has not been coupled to the magnetic particle 61 is removed from the cuvette 20.

Then, as shown in (6) of FIG. 4, a substrate injection process is performed in which a substrate solution including an enzyme 66, which is a light-emitting substrate, is dispensed into the cuvette 20 and is then stirred again. Then, the cuvette 20 is transferred to the enzyme reaction table 30 by the first cuvette transferring system 32. After a predetermined reaction time required for enzyme reaction has elapsed, the cuvette 20 is transferred to the photometric system 31 by the second cuvette transferring system 33. When the enzyme 66 and the immune complex 67 are coupled to each other by the enzyme reaction, light Ls is emitted from the immune complex 67. In this case, a measurement process is performed in which the photometric system 31 measures the light Ls emitted from the cuvette. In a general analysis operation, in order to detect the amount of antigen, which is an analysis target, in the specimen, the following process is performed: an antigen, a magnetic particle, and an indicator antibody are coupled to each other to generate the immune complex 67; the immune complex reacts with an enzyme to emit light; the amount of light emitted is measured; and the analyzing unit 44 calculates the amount of antigen based on the measured amount of light.

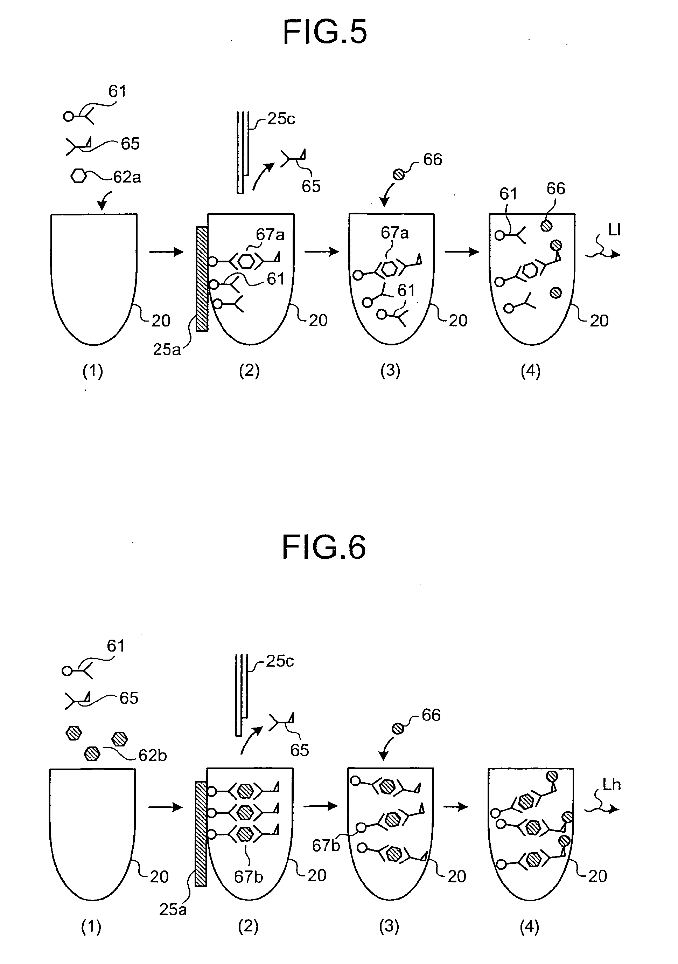

Similar to the antigen 62, which is a test target shown in FIG. 4, the low-concentration reagent contains a low concentration of antigen that couples to the magnetic particle 61 and the indicator antibody 65. Therefore, when substantially the same analysis process as that for the antigen 62 shown in FIG. 4 is performed, the antigen in the low-concentration reagent can emit light. A low-concentration reagent measuring process shown in FIG. 3 will be described with reference to FIG. 5.

As shown in (1) of FIG. 5, the specimen dispensing/transferring system 23, the first reagent dispensing/transferring system 28, and the second reagent dispensing/transferring system 29 dispense the magnetic particle 61, the indicator antibody 65, and a low-concentration reagent containing, for example, 0.3 ppm of antigen 62a into the cuvette 20. In the low-concentration reagent measuring process, in order to acquire the amount of light emitted corresponding to 0.3 ppm of antigen 62a, an analysis process of causing the antigen 62a contained in the low-concentration reagent in the cuvette 20 to emit light is performed to measure the amount of light emitted. Specifically, in the low-concentration reagent measuring process, the low-concentration reagent, the magnetic particle 61, and the indicator antibody 65 are dispensed into the cuvette 20 and are then stirred. Then, after a predetermined reaction time has elapsed, as shown in (2) of FIG. 5, the magnetic particle 61, the antigen 62a in the low-concentration reagent, and the indicator antibody 65 react with each other to generate an immune complex 67a.

Then, as shown in (2) of FIG. 5, in the low-concentration reagent measuring process, similar to a general analysis operation, the BF cleaning process is performed to remove the indicator antibody 65 that has not been coupled to the antigen 62a. Then, as shown in (3) of FIG. 5, in the low-concentration reagent measuring process, similar to a general analysis operation, a substrate solution containing the enzyme 66 is injected into the cuvette. In this case, as shown in (4) of FIG. 5, similar to the immune complex 67 shown in (6) of FIG. 4, the immune complex 67a is coupled to the enzyme 66 by enzyme reaction and light L1 of the amount corresponding to the amount of antigen 62a is emitted. In the low-concentration reagent measuring process, a measurement process is performed in which the photometric system 31 measures the light L1 emitted from the immune complex 67a.

Then, as shown in FIG. 3, after the low-concentration reagent measuring process (Step S22) is completed, the process control unit 42 controls each component of the measurement system 2 to perform a high-concentration reagent measuring process in which an analysis process of causing an antigen to emit light is performed on the high-concentration reagent and the amount of light emitted is measured, in order to acquire the performance of the high-concentration reagent (Step S24). Similar to the antigen 62, which is a test target shown in FIG. 4, the high-concentration reagent contains, for example, 1,000,000 ppm of antigen that couples to the magnetic particle 61 and the indicator antibody 65. Therefore, when substantially the same analysis process as that for the antigen 62 shown in FIG. 4 is performed, the antigen in the high-concentration reagent can emit light. Next, the high-concentration reagent measuring process shown in FIG. 3 will be described with reference to FIG. 6.

In the high-concentration reagent measuring process, first, as shown in (1) of FIG. 6, the specimen dispensing/transferring system 23, the first reagent dispensing/transferring system 28, and the second reagent dispensing/transferring system 29 dispense the magnetic particle 61, the indicator antibody 65, and a high-concentration reagent containing, for example, 1,000,000 ppm of antigen 62b into the cuvette. In the high-concentration reagent measuring process, in order to acquire the amount of light emitted corresponding to 1,000,000 ppm of antigen 62b, an analysis process of causing the antigen 62b contained in the high-concentration reagent in the cuvette 20 to emit light is performed to measure the amount of light emitted. Specifically, in the high-concentration reagent measuring process, the high-concentration reagent, the magnetic particle 61, and the indicator antibody 65 are dispensed into the cuvette 20 and are then stirred. Then, after a predetermined reaction time has elapsed, as shown in (2) of FIG. 6, the magnetic particle 61, the antigen 62b in the high-concentration reagent, and the indicator antibody 65 react with each other to generate an immune complex 67b.

Then, as shown in (2) of FIG. 6, in the high-concentration reagent measuring process, similar to a general analysis operation, the BF cleaning process is performed to remove the indicator antibody 65 that has not been coupled to the antigen 62b. Then, as shown in (3) of FIG. 6, in the high-concentration reagent measuring process, similar to a general analysis operation, a substrate solution containing the enzyme 66 is injected into the cuvette. In this case, as shown in (4) of FIG. 6, similar to the immune complex 67 shown in (6) of FIG. 4, the immune complex 67b is coupled to the enzyme 66 by enzyme reaction and light Lh of the amount corresponding to the amount of antigen 62b is emitted. In the high-concentration reagent measuring process, a measurement process is performed in which the photometric system 31 measures the light Lh emitted from the immune complex 67b.

Then, after the high-concentration reagent measuring process (Step S24) is completed, the reagent evaluating unit 46 calculates the concentration ratio of the low-concentration reagent and the high-concentration reagent based on the measurement result in the low-concentration reagent measuring process (Step S22) and the measurement result in the high-concentration reagent measuring process (Step S24) (Step S26). The measurement result in the low-concentration reagent measuring process (Step S22) is the amount of light L1 corresponding to the amount of antigen contained in the low-concentration reagent, and the measurement result in the high-concentration reagent measuring process (Step S24) is the amount of light Lh corresponding to the amount of antigen contained in the high-concentration reagent. When the amount of light emitted from the antigens 62a and 62b is proportional to the concentration thereof, the reagent evaluating unit 46 may use the ratio of the value measured in the low-concentration reagent measuring process and the value measured in the high-concentration reagent measuring process as the concentration ratio of the low-concentration reagent and the high-concentration reagent, without converting the amount of light into a concentration value. In contrast, when the amount of light emitted from the antigens 62a and 62b is not proportional to the concentration thereof, the reagent evaluating unit 46 may convert the measured value of the amount of light into a concentration value using a calibration curve of an analyzer 1, which has already been corrected by a calibrator, and calculate the concentration ratio.

Then, the reagent evaluating unit 46 determines whether the calculated concentration ratio is within a predetermined allowable range (Step S28). The allowable range is set based on the concentrations of the low-concentration reagent and the high-concentration reagent required to accurately specify an error in the error specifying process.

In the measurement process for acquiring a reference value in the measurement process for specifying an error, the measurement result obtained by the use of the low-concentration reagent is acquired as the reference value. In each measurement process for specifying an error in the measurement process for specifying an error, the measurement result obtained by the use of the high-concentration reagent is acquired as a measured value for error specification. In the error specifying process, the measured value for error specification is compared with the reference value. If the measured value for error specification is more than a predetermined multiple of the reference value, the removal error of the BF cleaning process or the cleaning error of the dispensing system, which is an error specification target, is specified. That is, in the error specifying process, it is possible to specify whether there is an error based on whether the ratio of the reference value and the measured value for error specification is within a predetermined allowable range.

Therefore, the ratio of the antigen concentration of the low-concentration reagent, which is the origin of the reference value, and the antigen concentration of the high-concentration reagent, which is the origin of the measured value for error specification, needs to be maintained at a concentration ratio suitable to accurately specify an error in the error specifying process. However, in some cases, the active states of the low-concentration reagent and the high-concentration reagent are changed over time after opening, which results in a variation in the performance required for measurement for error specification, that is, the antigen concentration. In Step S28, the reagent evaluating unit 46 determines whether the concentrations of the low-concentration reagent and the high-concentration reagent has varied, using a predetermined allowable range.

Concerning the allowable range of the concentration ratio, a case in which the amount of light emitted from the antigens 62a and 62b is proportional to the concentration will be described. For example, when the amount of light is 10 counts during the manufacture of the low-concentration reagent and the amount of light is 1,000,000 counts during the manufacture of the high-concentration reagent, and the active states of the low-concentration reagent and the high-concentration reagent do not vary, the ratio of the amount of light emitted from the low-concentration reagent and the amount of light emitted from the high-concentration reagent is 1:100,000.

However, when the active state of the low-concentration reagent has varied and the amount of light emitted therefrom is reduced, the high-concentration regent becomes dominant in the ratio of the amount of light emitted from the low-concentration reagent and the amount of light emitted from the high-concentration reagent. In the error specifying process, the specifying unit 45 specifies an error when the measured value for error specification is more than a predetermined multiple of the reference value. Therefore, when the active state of the low-concentration reagent varies and the amount of light emitted therefrom is reduced, the reference value for error specification is also reduced. Even when there is no error, the measured value for error specification can exceed a predetermined multiple of the reduced reference value. In this case, there is a concern that the specifying unit 45 erroneously determines that there is an error. Therefore, the upper limit of the allowable range of the ratio of the amounts of light emitted is set to, for example, 1:110,000, considering the allowable carry-over value of the analyzer 1, the measurement accuracy of the photometric system 31, and the dispensing accuracy of each dispensing system, in order to prevent an error in the determination of the specifying unit 45.

When the active state of the high-concentration reagent has varied and the amount of light emitted therefrom is reduced, the low-concentration regent becomes dominant in the ratio of the amount of light emitted from the low-concentration reagent and the amount of light emitted from the high-concentration reagent. Therefore, when the active state of the high-concentration reagent varies and the amount of light emitted therefrom is reduced, the measured value for error specification is also reduced. Even when there is an error, the reduced measured value for error specification can fall below a predetermined multiple of the reference value. In this case, there is a concern that the specifying unit 45 erroneously determines that there is no error. Therefore, the lower limit of the allowable range of the ratio of the amounts of light emitted is set to, for example, 1:90,000, considering the allowable carry-over value of the analyzer 1, the measurement accuracy of the photometric system 31, and the dispensing accuracy of each dispensing system, in order to prevent an error in the determination of the specifying unit 45.

The reagent evaluating unit 46 performs the determination process of Step S28 using the set allowable range. Then, if it is determined that the concentration ratio calculated in Step S26 is within a predetermined allowable range (Step S28: Yes), the reagent evaluating unit 46 considers that the concentration ratio of the low-concentration reagent and the high-concentration reagent is maintained at a value suitable to accurately specify an error in the error specifying process. Therefore, the reagent evaluating unit 46 evaluates that both the low-concentration reagent and the high-concentration reagent are normal (Step S30). Then, the reagent evaluating unit 46 determines that both the low-concentration reagent and the high-concentration reagent can be used (Step S32). When receiving the determination result for the use of the reagents from the reagent evaluating unit 46, the process control unit 42 and the specifying unit 45 perform the measurement process for error specification (Step S12) and the error specifying process (Step S14) using both the low-concentration reagent and the high-concentration reagent. The analyzer 1 performs the measurement process for error specification (Step S12) using the reagent that is evaluated to be normal, and specifies whether there is an error in the error specifying process (Step S14) based on the result of the measurement process for error specification.

Conversely, if it is determined that the concentration ratio calculated in Step S26 is not within the predetermined allowable range (Step S28: No), the reagent evaluating unit 46 considers that the concentration of at least one of the low-concentration reagent and the high-concentration reagent is not maintained at a value suitable to accurately specify an error in the error specifying process. Therefore, the reagent evaluating unit 46 evaluates that there is a defect in at least one of the low-concentration reagent and the high-concentration reagent (Step S34). Then, the reagent evaluating unit 46 determines that neither the low-concentration reagent nor the high-concentration reagent can be used (Step S36). When receiving the determination result for the use of the reagents from the reagent evaluating unit 46, the process control unit 42 and the specifying unit 45 cannot use both the low-concentration reagent and the high-concentration reagent, and the analyzer 1 outputs an error message indicating that there is a defect in at least one of the low-concentration reagent and the high-concentration reagent (Step S10).

As such, the analyzer 1 performs the measurement process for error specification (Step S12) using only the reagent that has been evaluated to be normal, and specifies whether there is an error in the error specifying process (Step S14) based on the result of the measurement process for error specification. Therefore, it is possible to prevent a specification error due to a denaturation of the reagent used, and it is possible to accurately specify an error.

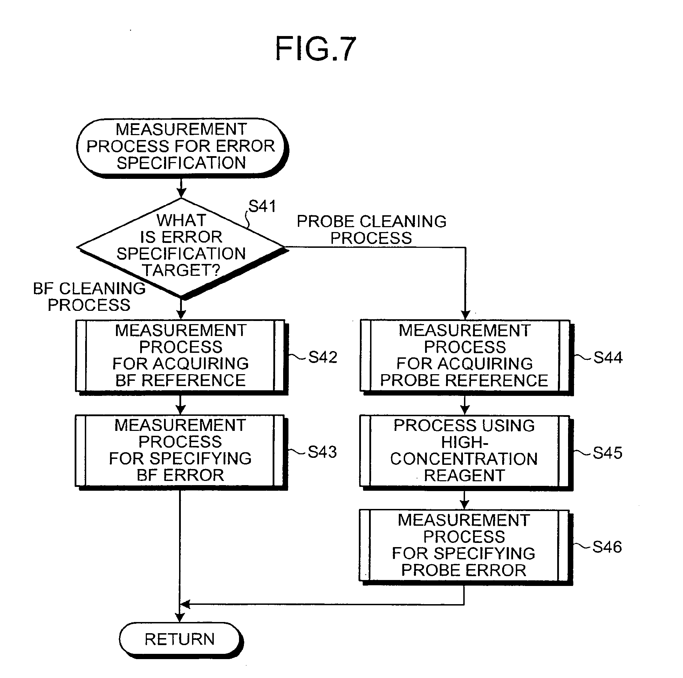

Next, the measurement process for error specification shown in FIG. 2 will be described with reference to FIG. 7. As shown in FIG. 7, the specifying unit 45 determines whether an error specification target is the BF cleaning process or the probe cleaning process in accordance with error specification information input from the input unit 43 (Step S41).

Then, if it is determined that an error specification target is the BF cleaning process (Step S41: BF cleaning process), the specifying unit 45 performs a measurement process for acquiring a BF reference (Step S42) and a measurement process for specifying a BF error (Step S43). Conversely, if it is determined that an error specification target is the probe cleaning process (Step S41: probe cleaning process), the specifying unit 45 performs a measurement process for acquiring a probe reference (Step S44), a process using the high-concentration reagent (Step S45), and a measurement process for specifying a probe error (Step S46).

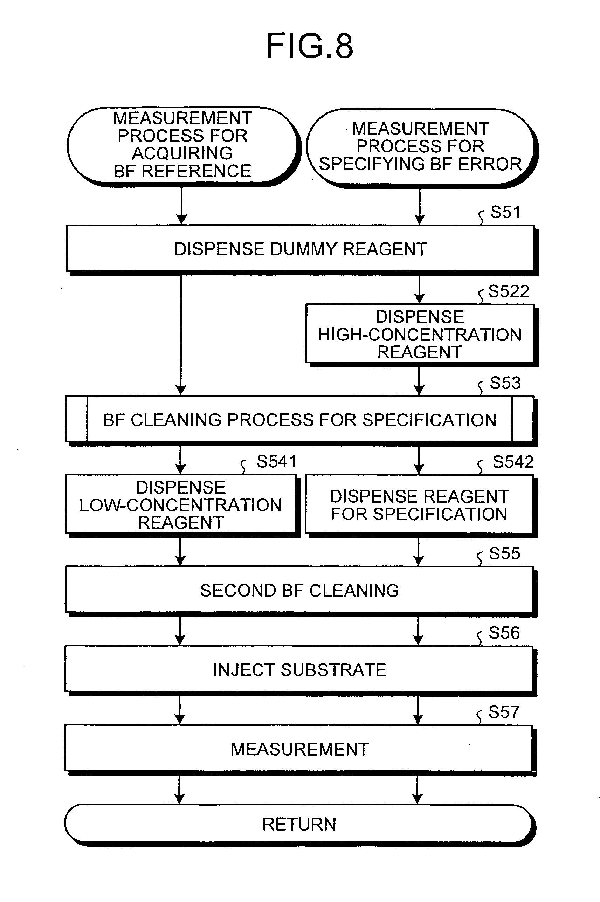

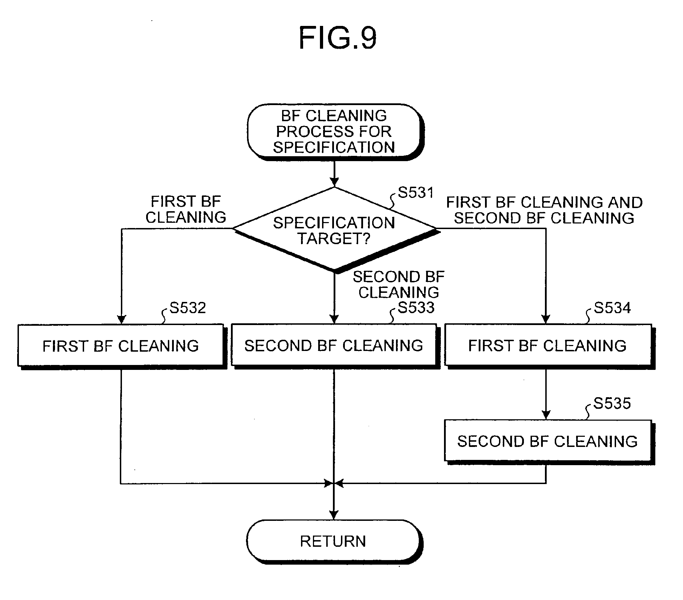

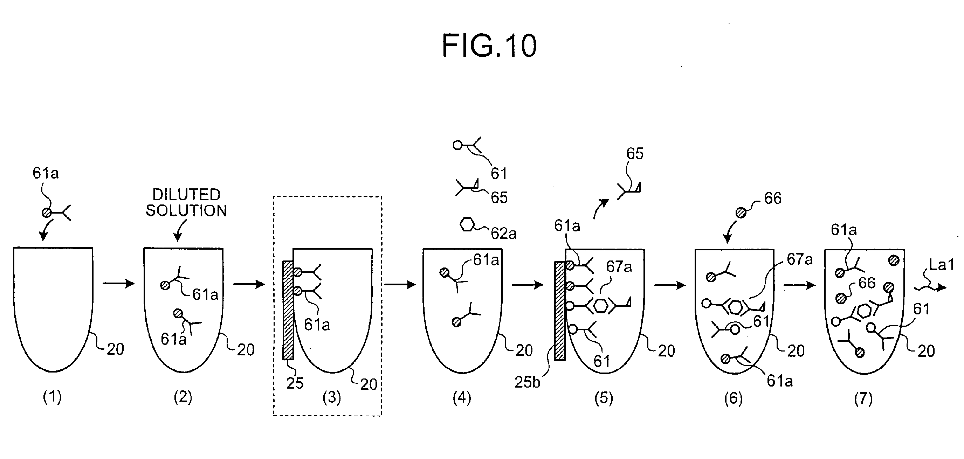

First, a case in which the error specification target is the BF cleaning process will be described in detail. First, the measurement process for acquiring a BF reference (Step S42) shown in FIG. 7 will be described with reference to FIGS. 8 to 10. FIG. 8 is a flowchart illustrating the procedure of the measurement process for acquiring a BF reference and the measurement process for specifying a BF error shown in FIG. 7. FIG. 9 is a flowchart illustrating the procedure of the BF cleaning process for specification shown in FIG. 8. FIG. 10 is a diagram illustrating the measurement process for acquiring a BF reference shown in FIG. 8.

As shown in FIG. 8 and (1) of FIG. 10, in the measurement process for acquiring a BF reference, instead of the first reagent dispensing process of a general analysis operation, a dummy reagent dispensing process is performed which dispenses a dummy reagent containing the magnetic particle 61a that does not react with the antigens in the low-concentration reagent and the high-concentration reagent (Step S51). Then, in the measurement process for acquiring a BF reference, as shown in (2) of FIG. 10, unlike the general analysis operation, the specimen dispensing process is not performed, but a diluted solution is injected in order to prevent the magnetic particle 61a dispersed in the dummy reagent dispensing process from being dried.

Then, in the measurement process for acquiring a BF reference, as shown in (3) of FIG. 10, the BF cleaning process for specification is performed (Step S53). In the BF cleaning process for specification, the BF cleaning process, which is a removal error specification target, is performed in accordance with the process pattern determined by the control unit 41. In the BF cleaning process for specification, as shown in FIG. 9, the process control unit 42 determines whether a specification target is the first BF cleaning process, the second BF cleaning process, or the first and second BF cleaning processes (Step S531). If it is determined that the specification target is the first BF cleaning process (Step S531: first BF cleaning), the process control unit 42 controls each component of the measurement system 2 to perform the first BF cleaning process using the magnetic particle collection system 25a and the BF cleaning nozzle 25c (Step S532). If it is determined that the specification target is the second BF cleaning process (Step S531: second BF cleaning), the process control unit 42 controls each component of the measurement system 2 to perform the second BF cleaning process using the magnetic particle collection system 25b and the BF cleaning nozzle 25d (Step S533). If it is determined that the specification target is the first and second BF cleaning processes (Step S531: first BF cleaning and second BF cleaning process), the process control unit 42 controls each component of the measurement system 2 to sequentially perform the first BF cleaning process (Step S534) and the second BF cleaning process (Step S535).

Then, in the measurement process for acquiring a BF reference, as shown in (4) of FIG. 10, a low-concentration reagent dispensing process is performed which dispenses the indicator antibody 65, the low-concentration reagent, and the magnetic particle 61 that can react with the antigen 62a in the low-concentration reagent (Step S541). In the measurement process for acquiring a BF reference, in order to acquire the amount of light emitted corresponding to 0.3 ppm of antigen 62a as the reference value, after the low-concentration reagent is dispensed, an analysis process of causing the antigen 62a in the low-concentration reagent to emit light is performed to measure the amount of light emitted. Specifically, in the measurement process for acquiring a BF reference, the low-concentration reagent is dispensed into the cuvette 20 and is then stirred. Then, after a predetermined reaction time has elapsed, as shown in (5) of FIG. 10, the magnetic particle 61, the antigen 62a in the low-concentration reagent, and the indicator antibody 65 react with each other to generate an immune complex 67a. Then, in the measurement process for acquiring a BF reference, similar to a general analysis operation, the second BF cleaning process is performed (Step S55), and the indicator antibody 65 that has not been coupled to the magnetic particle carrier is removed. Incidentally, the immune complex 67a is collected by the magnetic particle collection system 25b of the BF table 25 and is not removed from the cuvette 20.

Then, as shown in (6) of FIG. 10, in the measurement process for acquiring a BF reference, similar to a general analysis operation, a substrate injection process is performed in which a substrate solution containing the enzyme 66 is injected into the cuvette 20 (Step S56). In this case, as shown in (7) of FIG. 10, similar to the immune complex 67 shown in (6) of FIG. 4, the immune complex 67a is coupled to the enzyme 66 by enzyme reaction to emit light La1. In the measurement process for acquiring a BF reference, a measurement process is performed in which the photometric system 31 measures the light La1 emitted from the immune complex 67a (Step S57). In this way, it is possible to obtain the amount of light emitted as the reference value.

Even when 0.3 ppm of antigen is contained, the analyzer 1 can output the measurement result of another antigen, which is an analysis target, without any clinical problem. Therefore, the amount of light La1 measured as the reference value of BF cleaning in the measurement process for acquiring a BF reference is a standard for determining impurity concentration that enables the analyzer 1 to output the measurement result without any clinical problem.

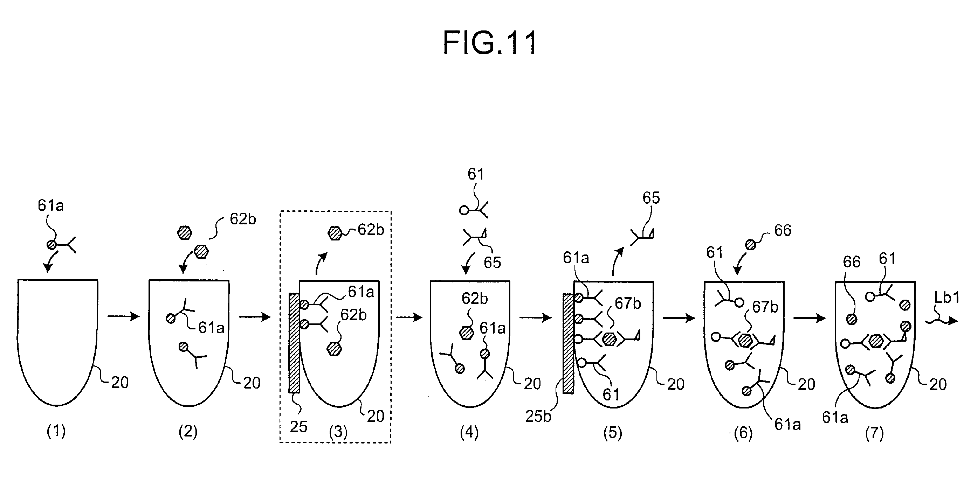

Next, the measurement process for specifying a BF error will be described with reference to FIGS. 8 and 11.

FIG. 11 is a diagram illustrating the measurement process for specifying a BF error shown in FIG. 8. As shown in FIG. 8 and (1) of FIG. 11, in the measurement process for specifying a BF error, similar to the measurement process for acquiring a BF reference, a dummy reagent dispensing process is performed which dispenses a dummy reagent containing the magnetic particle 61a (Step S51).

Then, in the measurement process for specifying a BF error, as shown in (2) of FIG. 11, instead of the specimen dispensing process of a general analysis operation, a high-concentration reagent dispensing process is performed in which a high-concentration reagent containing the high-concentration antigen 62b is dispensed into the cuvette 20 (Step S522). For example, the high-concentration reagent contains 1,000,000 ppm of antigen 62b.

Then, in the measurement process for specifying a BF error, as shown in (3) of FIG. 11, similar to the BF cleaning process for specification that is performed in the measurement process for acquiring a reference value, the procedure shown in FIG. 9 is performed and the BF cleaning process for specification is performed (Step S53). That is, in the measurement process for specifying a BF error, the first BF cleaning process is performed, when the first BF cleaning process is performed as the BF cleaning process for specification in the measurement process for acquiring a BF reference, and the second BF cleaning process is performed, when the second BF cleaning process is performed as the BF cleaning process for specification in the measurement process for acquiring a reference value. In addition, both the first BF cleaning process and the second BF cleaning process are performed, when both the first BF cleaning process and the second BF cleaning process are performed as the BF cleaning process for specification in the measurement process for acquiring a reference value.

When the BF cleaning process is performed normally, the antigen 62b in the high-concentration reagent is removed from the cuvette 20 such that there is no remaining antigen in the cuvette. However, for example, when the BF cleaning nozzle 25c or the BF cleaning nozzle 25d is clogged, a cleaning solution is not normally discharged, or the liquid and the cleaning solution in the cuvette 20 are not normally sucked. When a BF cleaning error occurs due to, for example, the clogging of the BF cleaning nozzle, as shown in (3) of FIG. 11, the antigen 62b remains in the cuvette 20.

In order to specify the BF cleaning error, in the measurement process for specifying a BF error, after the BF cleaning process for specification is performed, an analysis process in which the antigen 62b remaining in the cuvette 20 can emit light is performed, and the amount of light emitted from the antigen 62b remaining in the cuvette 20 is measured as a measured value for error specification with respect to BF cleaning. Specifically, in the measurement process for specifying a BF error, as shown in (4) of FIG. 11, a specifying reagent dispensing process is performed which dispenses a specifying reagent containing the indicator antibody 65 and the magnetic particle 61 (Step S542). Then, the reagent is stirred in the cuvette 20. Then, after a predetermined reaction time has elapsed, as shown in (5) of FIG. 11, the magnetic particle 61, the remaining antigen 62b, and the indicator antibody 65 are coupled to generate the immune complex 67b. Then, in the measurement process for specifying a BF error, similar to a general analysis operation, the second BF cleaning process is performed (Step S55) to remove the indicator antibody 65 that has not been coupled to the magnetic particle carrier. In addition, the immune complex 67b is collected by the magnetic particle collection system 25b of the BF table 25 and is not removed from the cuvette 20.

Then, as shown in (6) of FIG. 11, in the measurement process for specifying a BF error, similar to a general analysis operation, a substrate injection process is performed in which a substrate solution containing the enzyme 66 is injected into the cuvette 20 (Step S56). In this case, as shown in (7) of FIG. 11, similar to the immune complex 67 shown in (6) of FIG. 4, the immune complex 67b is coupled to the enzyme 66 by enzyme reaction to emit light Lb1. In the measurement process for specifying a BF error, a measurement process is performed in which the photometric system 31 measures the light Lb1 emitted from the immune complex 67b as a measured value for error specification (Step S57). The amount of light Lb1 measured as the measured value for error specification corresponds to the amount of antigen 62b remaining after the BF cleaning process for specification is performed.

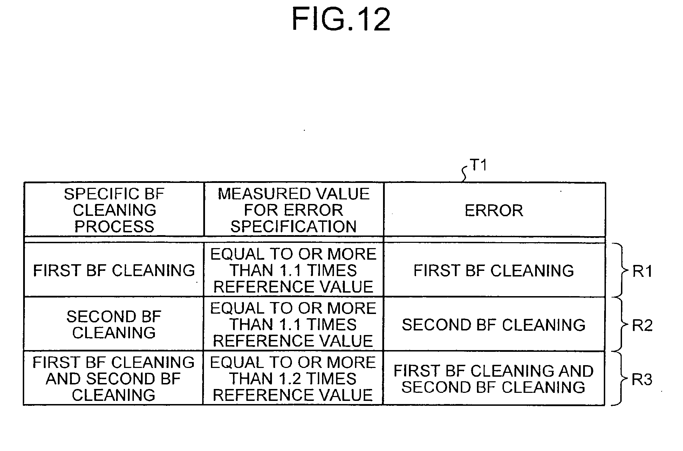

Next, concerning the error specifying process shown in FIG. 2, a case in which it is specified whether there is an error in the BF cleaning process will be described. When the measured value for error specification acquired in the measurement process for specifying a BF error is not within the allowable range that is set based on the reference value acquired in the measurement process for acquiring a BF reference, the specifying unit 45 specifies that there is a removal error in a specific BF cleaning process. The specifying unit 45 performs an error specifying process while referring to a table T1 shown in FIG. 12, which is stored in the storage unit 47, as a predetermined allowable range. The reference value obtained in the measurement process for acquiring a BF reference is the amount of light La1 emitted when 0.3 ppm of antigen 62a is contained, and is a standard for determining impurity concentration that enables the analyzer to output the measurement result of another antigen, which is an analysis target, without any clinical problem. Therefore, the table T1 includes a criterion for the reference value of a specific BF cleaning process.

An error specifying process when the first BF cleaning process is performed as a specific BF cleaning process in the measurement process for acquiring a BF reference and the measurement process for specifying a BF error will be described. When the first BF cleaning process is performed as a specific BF cleaning process and the measured value for error specification is less than 1.1 times the reference value, which is the amount of light emitted from 0.3 ppm of antigen 62a, it is possible to output the measurement result of the antigen, which is an analysis target, without any clinical problem. Therefore, when the measured value for error specification is less than 1.1 times the reference value, the specifying unit 45 considers that it is possible to remove the antigen 62b in the high-concentration reagent such that a clinical problem does not arise in the first BF cleaning process. Therefore, the specifying unit 45 determines that there is no removal error due to the clogging of the BF cleaning nozzle. In contrast, as shown in a row R1 of the table T1, when the measured value for error specification is equal to or more than 1.1 times the reference value, the specifying unit 45 determines that the antigen 62b in the high-concentration reagent is not sufficiently removed in the first BF cleaning process such that the measurement result is affected by the insufficient removal of the antigen 62b and a removal error occurs due to, for example, the clogging of the BF cleaning nozzle 25c.

When the second BF cleaning process is performed as a specific BF cleaning process in the measurement process for acquiring a BF reference and the measurement process for specifying a BF error and the measured value for error specification is less than 1.1 times the reference value, it is possible to output the measurement result of the antigen, which is an analysis target, without any clinical problem. Therefore, when the measured value for error specification is less than 1.1 times the reference value, the specifying unit 45 determines that there is no removal error due to, for example, the clogging of the BF cleaning nozzle in the second BF cleaning process. In contrast, as shown in a row R2 of the table T1, when the measured value for error specification is equal to or more than 1.1 times the reference value, the specifying unit 45 determines that the antigen 62b in the high-concentration reagent is not sufficiently removed in the second BF cleaning process such that the measurement result is clinically affected by the insufficient removal of the antigen 62b and a removal error occurs due to, for example, the clogging of the BF cleaning nozzle 25d.

When the first BF cleaning process and the second BF cleaning process are performed as a specific BF cleaning process in the measurement process for acquiring a BF reference and the measurement process for specifying a BF error and the measured value for error specification is less than 1.2 times the reference value, it is possible to output the measurement result of the antigen, which is an analysis target, without any clinical problem. Therefore, when the measured value for error specification is less than 1.2 times the reference value, the specifying unit 45 determines that there is no removal error due to, for example, the clogging of the BF cleaning nozzle in the first BF cleaning process and the second BF cleaning process. In contrast, as shown in a row R3 of the table T1, when the measured value for error specification is equal to or more than 1.2 times the reference value, the specifying unit 45 determines that the antigen 62b in the high-concentration reagent is not sufficiently removed in the first BF cleaning process and/or the second BF cleaning process such that the measurement result is clinically affected by the insufficient removal of the antigen 62b and a removal error occurs due to, for example, the clogging of the BF cleaning nozzles 25c and 25d.

Furthermore, the analyzer 1 measures the amount of light emitted from the high-concentration reagent that is actually used, in the reagent evaluating process (Step S6) before the measurement process for error specification (Step S12) is performed. Therefore, the specifying unit 45 can calculate a carry-over value of the BF cleaning process, which is an error specification target, based on a value obtained by dividing the measured value for error specification of the BF cleaning process, which is measured in the measurement process for specifying a BF error in the error specifying process (Step S14), by the measurement result obtained in the high-concentration reagent measuring process of the reagent evaluating process.

When the amount of light emitted from the antigens 62a and 62b is proportional to the concentration of the antigens, the measurement result in the high-concentration reagent measuring process is E31 counts, and the measured value for error specification in the measurement process for specifying a BF error is E41 counts, a carry-over value C in the BF cleaning process, which is an error specification target, can be calculated by Expression 1 given below.

$$C = (E41/E31) \times 10^6 \text{ [ppm]} \tag{1}$$

Specifically, when the measurement result in the high-concentration reagent measuring process is 800,000 counts and the measured value for error specification in the measurement process for specifying a BF error is 40 counts, the specifying unit 45 can calculate that the carry-over value of the BF cleaning process, which is an error specification target, is 50 [ppm] using Expression 1. The control unit 41 controls the output unit 48 to output the carry-over value calculated by the specifying unit 45. The operator can confirm the output result, and can recognize the carry-over value of the BF cleaning process, which is an error specification target, and take appropriate measures.

Conventionally, the operator of the analyzer calculates the carry-over value based on the measurement results obtained in each measurement process in order to acquire the carry-over value. As a result, there is a concern that a calculation error occurs. In contrast, with the analyzer, the analyzer 1 itself automatically calculates the carry-over value based on the measurement results obtained in each measurement process, and outputs the carry-over value. Therefore, according to the analyzer 1, the operator does not need to perform a complicated calculation process. Furthermore, according to the analyzer 1, since the operator does not need to calculate the carry-over value, it is possible to prevent the calculation error of the carry-over value due to manual calculation and output an accurate carry-over value.

As such, in order to specify the error of the BF cleaning process, in the measurement process for acquiring a BF reference and the measurement process for specifying a BF error, the analyzer 1 performs the processes other than the BF cleaning process, which is an error specification target, substantially in the same way. It is not necessary to consider the errors of other processes and it is possible to accurately verify only the removal error of the BF cleaning process. The analyzer 1 can specify whether there is an error in the BF cleaning process based on the measurement result measured by the photometric system 31. Therefore, it is not necessary to perform colorimetry using a spectrophotometer that is provided separately from the analyzer, unlike the conventional art. Therefore, the analyzer 1 can accurately and easily specify the error of the BF cleaning process.

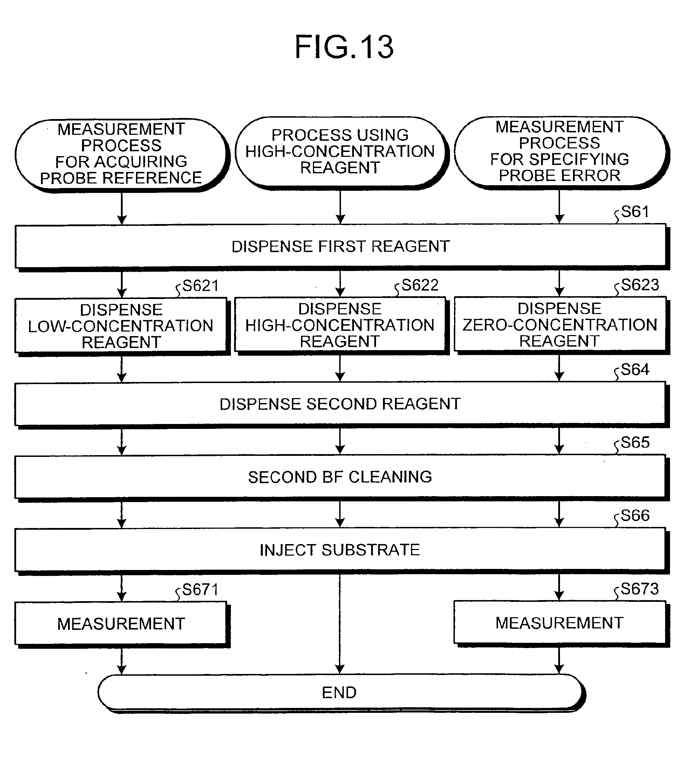

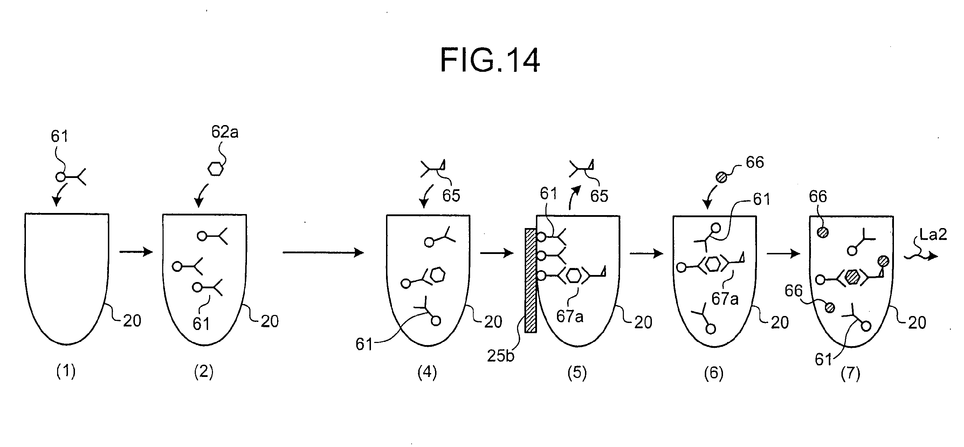

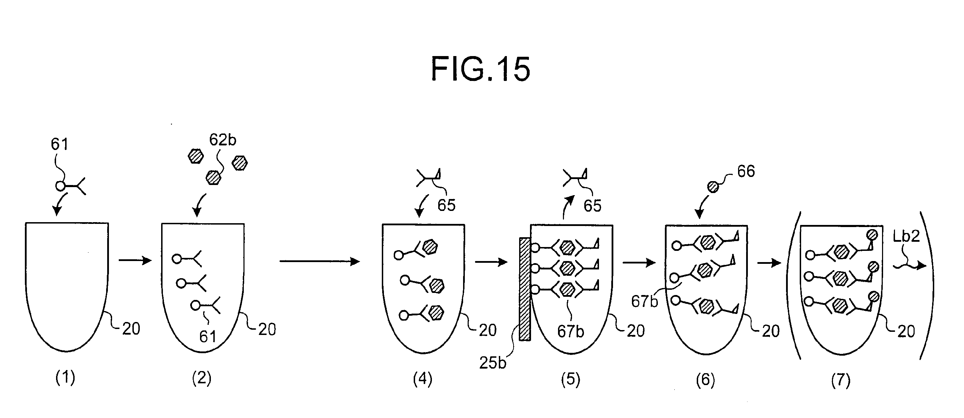

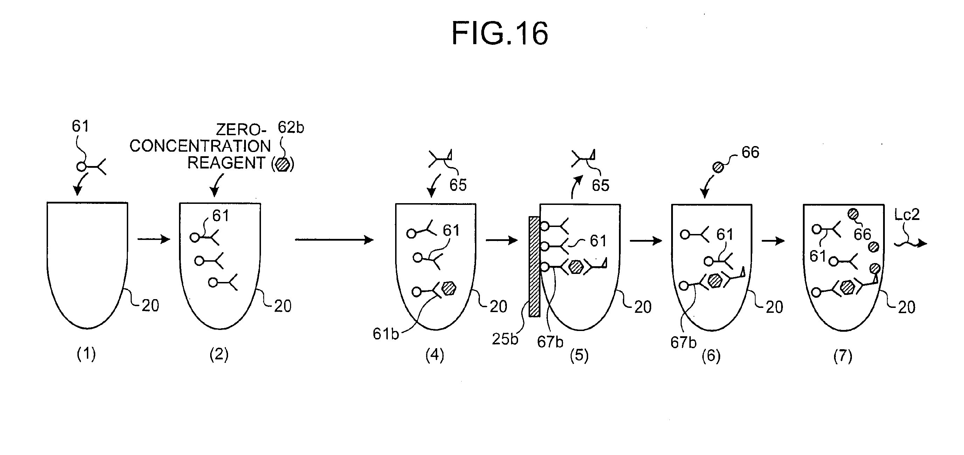

Next, a case in which an error specification target is a probe cleaning process will be described in detail. First, a measurement process for acquiring a probe reference, a process using a high-concentration reagent, and a measurement process for specifying a probe error shown in FIG. 7 will be described with reference to FIGS. 13 to 16. FIG. 13 is a diagram illustrating the procedure of the measurement process for acquiring a probe reference, the process using a high-concentration reagent, and the measurement process for specifying a probe error shown in FIG. 7. FIG. 14 is a diagram illustrating the measurement process for acquiring a probe reference shown in FIG. 13. FIG. 15 is a diagram illustrating the process using a high-concentration reagent shown in FIG. 13. FIG. 16 is a diagram illustrating the measurement process for specifying a probe error shown in FIG. 13.

First, the measurement process for acquiring a probe reference will be described. As shown in FIG. 13 and (1) of FIG. 14, in the measurement process for acquiring a probe reference, a first reagent dispensing process is performed which dispenses a first reagent containing the magnetic particle 61 that can react with the antigens contained in the low-concentration reagent and the high-concentration reagent (Step S61). Then, in the measurement process for acquiring a probe reference, as shown in (2) of FIG. 14, a low-concentration reagent dispensing process is performed which dispenses a low-concentration reagent containing 0.3 ppm of antigen 62a into the cuvette 20 using the probe of the dispensing/transferring system, which is a cleaning error specification target (Step S621). Then, the reagent in the cuvette 20 is stirred. After a predetermined reaction time has elapsed, the magnetic particle 61 is coupled to the antigen 62a in the low-concentration reagent to generate a magnetic particle carrier.

Even when 0.3 ppm of antigen 62a is contained, the analyzer 1 can output the measurement result of another antigen, which is an analysis target, without any clinical problem. Therefore, in the measurement process for acquiring a probe reference, in order to acquire the amount of light emitted from 0.3 ppm of antigen 62a as the reference value of the probe cleaning process, after the low-concentration reagent is dispensed, an analysis process in which the antigen 62a in the low-concentration reagent can emit light is performed to measure the amount of light emitted.

Specifically, in the measurement process for acquiring a probe reference, as shown in (4) of FIG. 14, after the low-concentration reagent dispensing process is performed, the second reagent dispensing process is performed in which the second reagent dispensing/transferring system 29 dispenses the second reagent containing the indicator antibody 65 into the cuvette 20 (Step S64). Then, the reagent in the cuvette 20 is stirred. After a predetermined reaction time has elapsed, as shown in (5) of FIG. 14, the magnetic particle carrier and the indicator antibody 65 are coupled to generate the immune complex 67a. Then, in the measurement process for acquiring a probe reference, as shown in (5) of FIG. 14, similar to a general analysis operation, the second BF cleaning process is performed (Step S65) to remove the indicator antibody 65 that has not been coupled to the magnetic particle carrier. The immune complex 67a is collected by the magnetic particle collection system of the BF table 25 and is not removed from the cuvette 20. Then, as shown in (6) of FIG. 14, in the measurement process for acquiring a probe reference, similar to a general analysis operation, a substrate injection process is performed in which a substrate solution containing the enzyme 66 is injected into the cuvette 20 (Step S66). In this case, as shown in (7) of FIG. 14, similar to the immune complex 67 shown in (6) of FIG. 4, the immune complex 67a is coupled to the enzyme 66 by enzyme reaction to emit light La2. In the measurement process for acquiring a probe reference, a measurement process is performed in which the photometric system 31 measures the light La2 emitted from the immune complex 67a (Step S671). In this way, it is possible to obtain the amount of light emitted as the reference value.

Next, the process using the high-concentration reagent will be described with reference to FIGS. 13 and 15. In the process using the high-concentration reagent, as shown in FIG. 13 and (1) of FIG. 15, similar to the measurement process for acquiring a probe reference, the first reagent dispensing process is performed which dispenses the first reagent containing the magnetic particle 61 (Step S61). Then, in the process using the high-concentration reagent, as shown in (2) of FIG. 15, a high-concentration reagent dispensing process is performed which dispenses a high-concentration reagent containing the high-concentration antigen 62b into the cuvette 20 using the probe of the dispensing/transferring system, which is a cleaning error specification target (Step S622). The high-concentration reagent contains, for example, 1,000,000 ppm of antigen 62b. The probe of the dispensing/transferring system that has dispensed the high-concentration reagent is cleaned before a liquid, which is the next dispensing target, is dispensed. Then, in the process using the high-concentration reagent, similar to the measurement process for acquiring a probe reference, the second reagent dispensing process shown in (4) of FIG. 15 (Step S64), the second BF cleaning process shown in (5) of FIG. 15 (Step S65), and the substrate injection process shown in (6) of FIG. 15 (Step S66) are performed. Incidentally, in the process using the high-concentration reagent, as shown in (7) of FIG. 15, the immune complex 67b and the enzyme 66 are coupled to emit light Lb2. The photometric system 31 ends the process using the high-concentration reagent without measuring the light.

Next, the measurement process for specifying a probe error will be described with reference to FIGS. 13 and 16. In the measurement process for specifying a probe error, as shown in FIG. 13 and (1) of FIG. 16, similar to the measurement process for acquiring a probe reference and the process using the high-concentration reagent, the first reagent dispensing process is performed which dispenses the first reagent containing the magnetic particle 61 (Step S61).

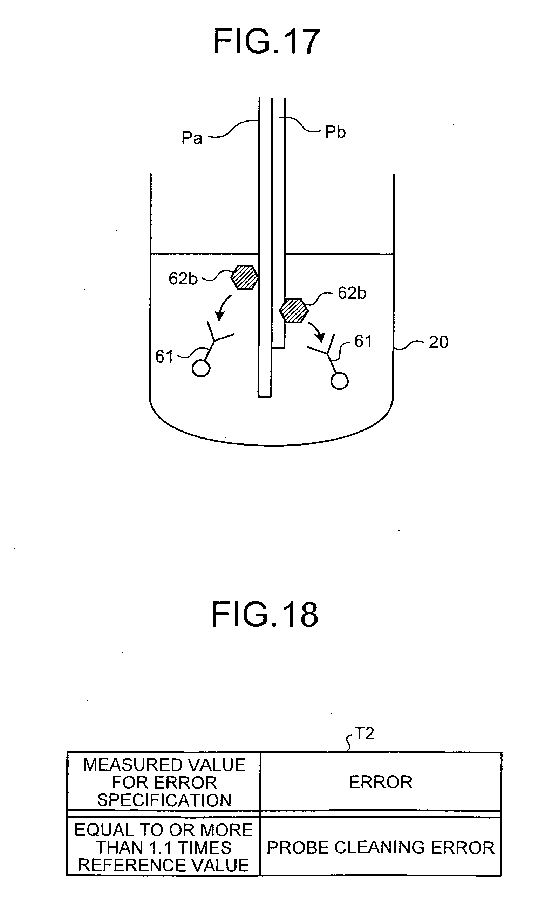

Then, in the measurement process for specifying a probe error, as shown in (2) of FIG. 16, a zero-concentration reagent dispensing process is performed which dispenses a zero-concentration reagent containing no antigen using the probe of the dispensing/transferring system, which is a cleaning error specification target (Step S623). As shown in FIG. 17, when probes Pa and Pb of the dispensing/transferring system are not sufficiently cleaned, in some cases, the antigen 62b in the high-concentration reagent that has been dispensed by the probes Pa and Pb before the zero-concentration reagent is dispensed is not sufficiently removed, but remains in the probes Pa and Pb. When the zero-concentration reagent is dispensed with the antigen 62b remaining in the probes Pa and Pb, the antigen 62b remaining in the probes Pa and Pb is mixed with the material in the cuvette 20. In order to specify the probe cleaning error, in the measurement process for specifying a probe error, after the zero-concentration reagent dispensing process is performed, an analysis process in which the antigen 62b that remains in the probes Pa and Pb and is then mixed with the material in the cuvette 20 can emit light is performed, and the amount of light emitted from the antigen 62b is measured as the measured value for error specification.

Specifically, in the measurement process for specifying a probe error, as shown in (4) of FIG. 16, after the zero-concentration reagent dispensing process is performed, the reagent in the cuvette 20 is stirred. After a predetermined reaction time has elapsed, the magnetic particle 61 and the antigen 62b that remains in the probes Pa and Pb and is then mixed with the material in the cuvette 20 are coupled to generate a magnetic particle carrier 61b. Then, the second reagent dispensing process is performed which dispenses the second reagent containing the indicator antibody 65 into the cuvette (Step S64). In this case, the reagent in the cuvette 20 is stirred, and after a predetermined reaction time has elapsed, the magnetic particle carrier 61b and the indicator antibody 65 are coupled to generate an immune complex 67b. Then, in the measurement process for specifying a probe error, as shown in (5) of FIG. 16, similar to the measurement process for acquiring a probe reference, the second BF cleaning process is performed (Step S65) to remove the indicator antibody 65 that has not been coupled to the magnetic particle carrier 61b. The immune complex 67b is collected by the magnetic particle collection system 25b of the BF table 25 and is not removed from the cuvette 20.

Then, as shown in (6) of FIG. 16, in the measurement process for specifying a probe error, similar to the measurement process for acquiring a probe reference, a substrate injection process is performed in which a substrate solution containing the enzyme 66 is injected into the cuvette 20 (Step S66). In this case, as shown in (7) of FIG. 16, similar to the immune complex 67 shown in (6) of FIG. 4, the immune complex 67b is coupled to the enzyme 66 by enzyme reaction to emit light Lc2. In the measurement process for specifying a probe error, a measurement process is performed in which the photometric system 31 measures the light Lc2 emitted from the immune complex 67b (Step S673). In this way, it is possible to obtain the amount of light emitted as the measured value for error specification of the probe cleaning process. The amount of light Lc2 measured as the measured value for error specification corresponds to the amount of antigen 62b that remains in the probe after the probe cleaning process, which is a cleaning error target, and is mixed with the material in the cuvette 20 during the next liquid dispensing process.

Next, the error specifying process shown in FIG. 2 when it is specified whether there is an error in the probe cleaning process will be described in detail. When the measured value for error specification acquired in the measurement process for specifying a probe error is not within the allowable range that is set based on the reference value acquired in the measurement process for acquiring a probe reference, the specifying unit 45 specifies that there is a cleaning error in the probe that dispenses the high-concentration reagent. The specifying unit 45 performs an error specifying process while referring to a table T2 shown in FIG. 18, which is stored in the storage unit 47, as a predetermined allowable range. The reference value obtained in the measurement process for acquiring a probe reference is the amount of light La2 emitted when 0.3 ppm of antigen 62a is contained, and is a standard for determining impurity concentration that enables the analyzer to output the measurement result of another antigen, which is an analysis target, without any clinical problem. Therefore, the table T2 includes a criterion for the reference value of the probe cleaning process.

When the measured value for error specification is less than 1.1 times the reference value, which is the amount of light emitted from 0.3 ppm of antigen 62a, the specifying unit 45 can output the measurement result of the antigen, which is an analysis target, without any clinical problem. Therefore, when the measured value for error specification is less than 1.1 times the reference value, the specifying unit 45 considers that the dispensing/transferring system dispensing the high-concentration reagent can clean and remove the antigen 62b in the high-concentration reagent attached to the probe such that no clinical problem arises. Therefore, the specifying unit 45 determines that there is no cleaning error in the dispensing/transferring system that dispenses the high-concentration reagent. In contrast, as shown in the table T2, when the measured value for error specification is equal to or more than 1.1 times the reference value, the specifying unit 45 considers that it is difficult for the dispensing/transferring system dispensing the high-concentration reagent to sufficiently remove the antigen 62b attached to the probe and there remains such an amount of antigen 62b that the measurement result is affected. Therefore, the specifying unit 45 determines that there is a probe cleaning error in the dispensing/transferring system that dispenses the high-concentration reagent.

Furthermore, similar to the process of specifying the error of the BF cleaning process, the specifying unit 45 can calculate a carry-over value of the probe cleaning process, which is an error specification target, based on a value obtained by dividing the measured value for specifying the error of the probe cleaning process, which is measured in the measurement process for specifying a probe error in the error specifying process (Step S14), by the measurement result measured in the high-concentration reagent measuring process of the reagent evaluating process.

When the amount of light emitted from the antigens 62a and 62b is proportional to the concentration, the measurement result in the high-concentration reagent measuring process is E32 counts, and the measured value for error specification in the measurement process for specifying a probe error is E42 counts, the carry-over value C of the BF cleaning process, which is an error specification target, can be calculated by Expression 2 given below:

$$C = (E42/E32) \times 10^6 \ [ppm] \qquad (2)$$

The control unit 41 controls the output unit 48 to output the carry-over value calculated by the specifying unit 45. The operator can confirm the output result, thereby recognize the carry-over value of the probe process, which is an error specification target, and take appropriate measures.

As such, in order to specify the error of the probe cleaning process, in the measurement process for acquiring a probe reference, the process using the high-concentration reagent, and the measurement process for specifying a probe error, the analyzer 1 determines whether the material in the liquid that has been dispensed previously is removed in the cleaning process for the dispensing/transferring system without any clinical problem, based on the measured value for error specification, which is the amount of light, obtained by sequentially dispensing the high-concentration reagent and the zero-concentration reagent using the dispensing/transferring system, which is a specification target. Therefore, the analyzer 1 performs the processes other than a liquid dispensing process of the dispensing/transferring system, which is an error specification target, substantially in the same way. It is not necessary to consider the errors of other processes and it is possible to accurately verify only the cleaning error of the dispensing/transferring system. The analyzer 1 can specify whether there is a cleaning error in the dispensing/transferring system based on the measurement result measured by the photometric system 31. Therefore, it is not necessary to perform colorimetry using a spectrophotometer that is provided separately from the analyzer, unlike the conventional art. Therefore, the analyzer 1 can accurately and easily specify the error of the probe cleaning process.

Further, the analyzer 1 evaluates the performances of the low-concentration reagent and the high-concentration reagent that are used to specify the errors of the BF cleaning process and the probe cleaning process, uses only the low-concentration reagent and the high-concentration reagent that are evaluated to be normal to perform each measurement process for error specification, and specifies whether an error occurs based on the result of the measurement process for error specification. Therefore, according to the first embodiment, it is possible to prevent an error from occurring in error specification due to the denaturation of the reagent used, and thus accurately perform error specification.

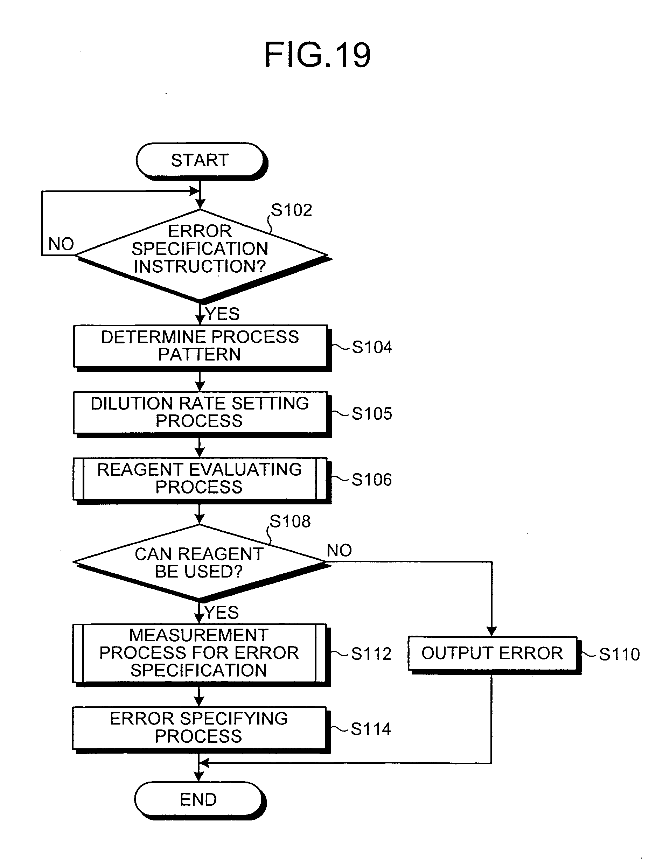

The analyzer 1 according to the first embodiment may dilute the high-concentration reagent such that the photometric system 31 can measure the high-concentration reagent according to the concentration value of the high-concentration reagent, and perform the high-concentration reagent measuring process. In this case, as shown in FIG. 19, the analyzer 1 performs the same procedure as that in Step S2 and Step S4 shown in FIG. 2 to perform a process of determining whether an instruction to specify an error is input (Step S102) and a process pattern determining process (Step S104). Then, the reagent evaluating unit 46 performs a dilution rate setting process that sets the dilution rate of the high-concentration reagent based on the measured value during the manufacture of the high-concentration reagent and the measurement range of the photometric system 31 (Step S105). When the analyzer 1 includes an automatic dilution system, the high-concentration reagent is diluted at the dilution rate set in Step S105 and is then used. In contrast, when the analyzer 1 does not include the automatic dilution system, the output unit 48 outputs the dilution rate set by the reagent evaluating unit 46, and the operator of the analyzer 1 dilutes the high-concentration reagent at the output dilution rate.

In general, a bar code storing information about the kind of reagent, a lot number, a bottle number, and the available period of a reagent, and the measured value of the amount of light emitted during manufacture as a value indicating the activation and concentration of the reagent is added to the reagent vessel containing the reagent used for the immune test. In general, when a reagent is used, the bar code attached to the reagent vessel is read to acquire information on the reagent. Therefore, it is possible to read the bar code attached to the reagent vessel containing the high-concentration reagent and acquire the indicated value of the high-concentration reagent in the analyzer 1.

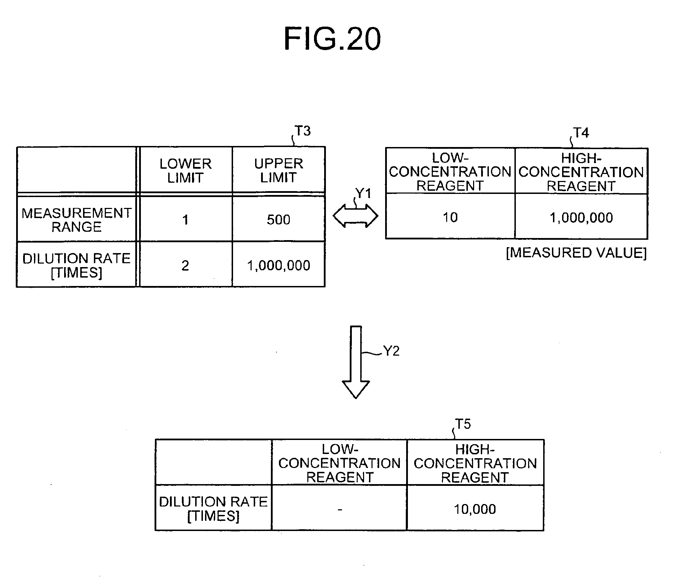

Next, the dilution rate setting process will be described in detail with reference to FIG. 20. As shown in a table T3 of FIG. 20, for example, the lower limit of the range in which the photometric system 31 can perform measurement, that is, the measurement range of the photometric system 31 is 1 count, and the upper limit thereof is 500 counts. For example, the lower limit of the dilution rate of the automatic dilution system of the analyzer 1 is 2 and the upper limit thereof is 1,000,000. Therefore, as shown in a table T4, the measured value of the amount of light emitted from the high-concentration reagent that is actually used during manufacture, that is, the indicated value thereof is 1,000,000 counts. In this case, as represented by an arrow Y1, the reagent evaluating unit 46 compares the measurement range, the dilution rate, and the measured value of the amount of light emitted from the high-concentration reagent, and sets the dilution rate of the high-concentration reagent to 10000, as represented by an arrow Y2. The amount of light emitted from the high-concentration reagent that is diluted at a dilution rate of 10000 is 100 counts even when the active state of the high-concentration reagent does not vary. Therefore, since the amount of light is within the measurement range of the photometric system 31, the photometric system 31 can reliably measure the amount of light.

Then, the measurement system 2 performs an analysis process in which the antigen in the high-concentration reagent that is diluted at the dilution rate set by the reagent evaluating unit 46 can emit light, and measures the amount of light emitted. That is, the analyzer 1 performs the same procedure as that in Step S6 shown in FIG. 2 using the high-concentration reagent that is diluted at the dilution rate set by the reagent evaluating unit 46, thereby performing the reagent evaluating process (Step S106). In this case, when calculating a concentration ratio based on the measured value measured in the high-concentration reagent measuring process, the reagent evaluating unit 46 uses a value obtained by multiplying the actually measured value by the dilution rate as the measured value of the original high-concentration reagent. Furthermore, the analyzer performs the same procedure as that in Steps S6 to S14 shown in FIG. 2 to perform a process of determining whether the reagent can be used (Step S108), an error output process (Step S110), and a measurement process for error specification (Step S112) and an error specifying process (Step S114). In the measurement process for error specification (Step S112), in order to calculate whether there is a carry-over, the high-concentration reagent containing a high concentration of antigen that is not diluted is used without any change.

When the high-concentration reagent is diluted and the high-concentration reagent measuring process (Step S24) is performed, the specifying unit 45 needs to multiply the measurement result measured in the high-concentration reagent measuring process by the dilution rate and to calculate a carry-over value using Expression 1 or 2, in the error specifying process (Step S114). For example, when the measurement result in the high-concentration reagent measuring process in which the high-concentration reagent is diluted at a dilution rate of 10000 is 80 counts and the measured value for error specification in the measurement process for specifying a BF error is 40 counts, the specifying unit 45 calculates that a carry-over value Cd is 50 [ppm], as represented by Expression 3 given below:

$$Cd = (40/(80 \times 10000)) \times 10^6 = 50 \ [ppm] \qquad (3)$$

As such, the analyzer 1 sets the optimal dilution rate of the high-concentration reagent based on the measurement range of the photometric system 31 and the measured value of the amount of light emitted during the manufacture of the high-concentration reagent. Therefore, when the analyzer 1 sets the dilution rate of the high-concentration reagent, the photometric system 31 can reliably perform a measurement process in the high-concentration reagent measuring process. Conventionally, a complicated process has been performed in which the operator of the analyzer manually calculates the dilution rate of the high-concentration reagent based on the indicated value of the high-concentration reagent. As a result, there is a concern that a calculation error occurs. In contrast, with the analyzer, the analyzer 1 itself automatically calculates and sets the optimal dilution rate of the high-concentration reagent. Therefore, according to the analyzer 1, the operator does not need to perform a complicated process. Furthermore, according to the analyzer 1, since the operator does not need to manually calculate the dilution rate, it is possible to prevent an error in the setting of the dilution rate, and thus output an accurate analysis result.

(Second embodiment)

Next, a second embodiment will be described. In the second embodiment, a case is described in which a zero-concentration reagent containing no antigen is used to individually calculate the concentrations of a low-concentration reagent and a high-concentration reagent and the low-concentration reagent and the high-concentration reagent are individually evaluated.

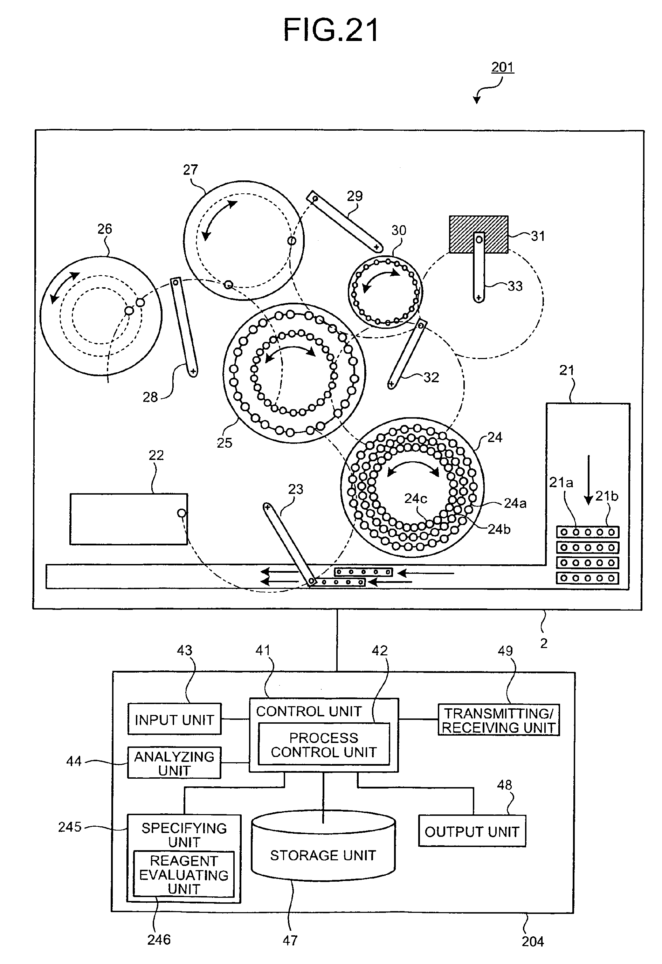

FIG. 21 is a diagram schematically illustrating the structure of an analyzer according to the second embodiment. As shown in FIG. 21, an analyzer 201 according to the second embodiment includes a specifying unit 245 having a reagent evaluating unit 246, instead of the specifying unit 45 of the control system 4 shown in FIG. 1. The reagent evaluating unit 246 evaluates the performances of the low-concentration reagent and the high-concentration reagent,

based on the measurement result obtained in an analysis process in which an antigen in the zero-concentration reagent can emit light and a zero-concentration reagent measuring process in which the photometric system 31 measures the amount of light emitted, in addition to the measurement results in the low-concentration reagent measuring process and the high-concentration reagent measuring process.

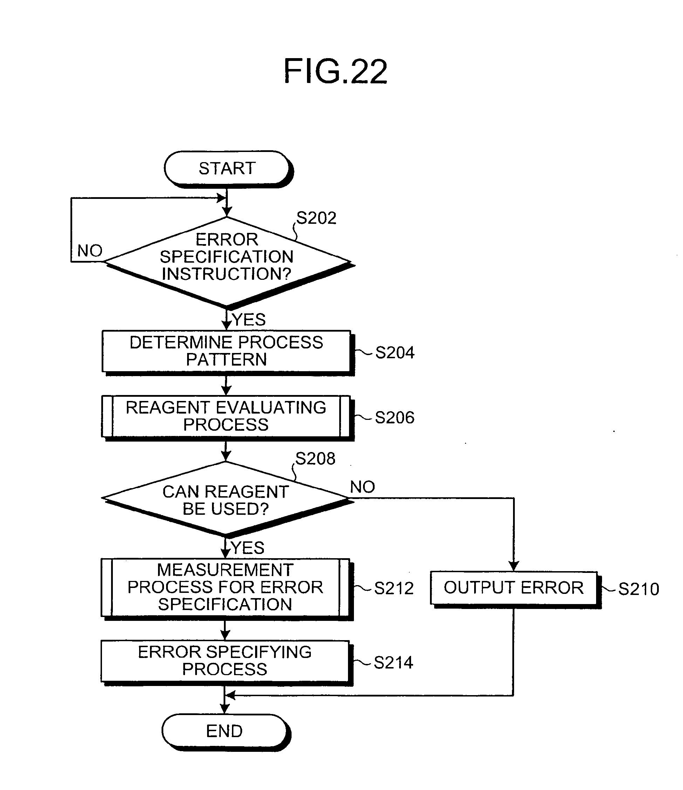

Next, the procedure of an error specifying process of the analyzer 201 shown in FIG. 21 will be described with reference to FIG. 22. As shown in FIG. 22, the analyzer 201 performs the same procedure as that in Step S2 and Step S4 shown in FIG. 2 to perform a process of determining whether an instruction to specify an error is input (Step S202) and a process pattern determining process (Step S204). Then, the reagent evaluating unit 246 performs a reagent evaluating process (Step S206). The reagent evaluating unit 246 calculates the actual concentrations of the low-concentration reagent and the high-concentration reagent based on the measurement result of the zero-concentration reagent in the reagent evaluating process and determines whether each of the low-concentration reagent and the high-concentration reagent can be used. The specifying unit 245 determines whether both the low-concentration reagent and the high-concentration reagent can be used based on the evaluation result of the reagent evaluating unit 246 (Step S208).

When the specifying unit 245 determines that the low-concentration reagent and high-concentration reagent cannot be used (Step S208: No), the output unit 48 outputs an error message indicating the reagents that cannot be used in the error specifying process (Step S210). The operator of the analyzer 1 can confirm the error message, recognize which reagent is defective, and take appropriate measures such as replacing only the defective reagent.

In contrast, when the specifying unit 245 determines that both the low-concentration reagent and the high-concentration reagent can be used (Step S208: Yes), each component of the measurement system 2 and the specifying unit 245 perform the same procedure as that in Step S12 and Step S14 shown in FIG. 2 using the low-concentration reagent and the high-concentration reagent that are evaluated to be available by the reagent evaluating unit 246 under the control of the process control unit 42, thereby performing a measurement process for error specification (Step S212) and an error specifying process (Step S214).

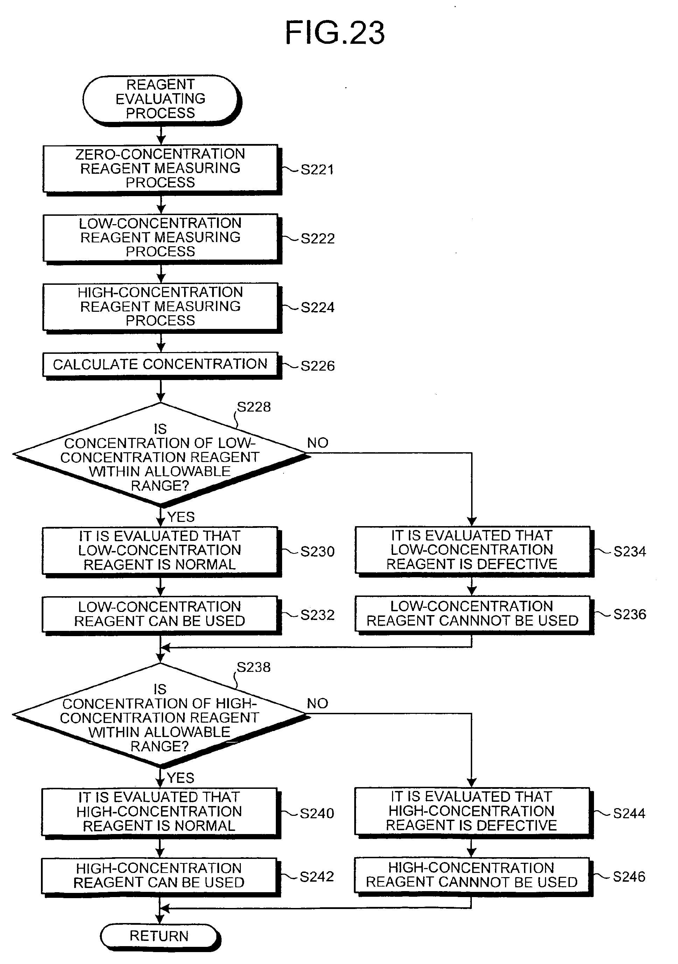

Next, the reagent evaluating process shown in FIG. 22 will be described with reference to FIG. 23. As shown in FIG. 23, the process control unit 42 controls each component of the measurement system 2 to perform a zero-concentration reagent measuring process in which an analysis process of causing the antigen to emit light is performed on the zero-concentration reagent and the amount of light emitted is measured, in order to acquire the measurement result of the zero-concentration reagent (Step S221).

In the zero-concentration reagent measuring process, the specimen dispensing/transferring system 23, the first reagent dispensing/transferring system 28, and the second reagent dispensing/transferring system 29 dispense the magnetic particle 61, the indicator antibody 65, and the zero-concentration reagent into the cuvette 20. In the zero-concentration reagent measuring process, after the magnetic particle 61, the indicator antibody 65, and the zero-concentration reagent are dispensed, they are stirred in the cuvette 20. Then, after a predetermined reaction time has elapsed, the BF cleaning process is performed, and a substrate solution containing the enzyme 66 is injected into the cuvette to perform enzyme reaction. Then, the photometric system 31 measures the amount of light emitted. The measurement result in the zero-concentration reagent measuring process becomes the reference value of each photometric system 31. The amount of light emitted from each specimen is obtained by adding the amount of light emitted indicating the actual concentration to the reference value.

Then, the analyzer 201 performs the same procedure as that in Step S22 and Step S24 shown in FIG. 3 to perform a low-concentration reagent measuring process (Step S222) and a high-concentration reagent measuring process (Step S224).

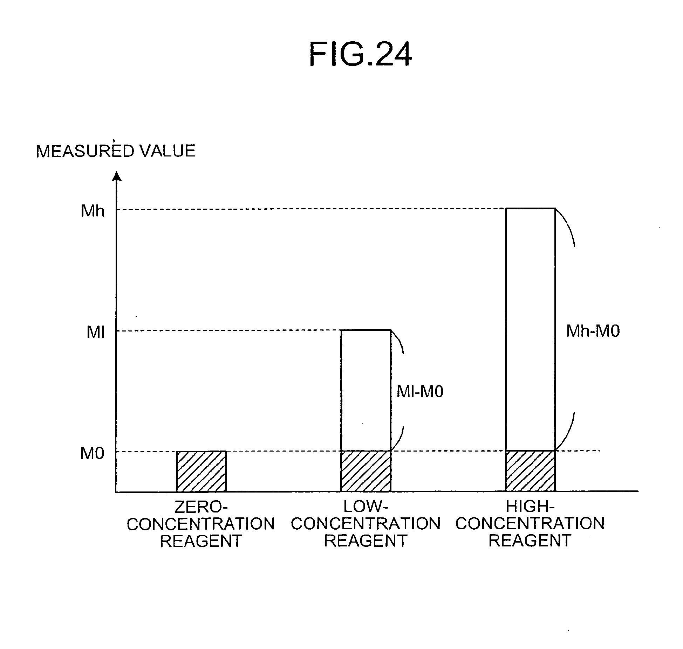

Then, the reagent evaluating unit 246 calculates the concentrations of the low-concentration reagent and the high-concentration reagent (Step S226). Specifically, as shown in FIG. 24, the reagent evaluating unit 246 subtracts a reference value M0, which is the measurement result in the zero-concentration reagent measuring process (Step S221), from a measured value M1 in the low-concentration reagent measuring process (Step S222). The difference value (M1-M0) is the amount of light emitted that corresponds to the original concentration of the low-concentration reagent. The reagent evaluating unit 246 calculates the actual concentration of the low-concentration reagent based on the difference value (M1-M0). Furthermore, the reagent evaluating unit 246 subtracts the reference value M0, which is the measurement result in the zero-concentration reagent measuring process (Step S221), from a measured value Mh in the high-concentration reagent measuring process (Step S224). The difference value (Mh-M0) is the amount of light emitted that corresponds to the original concentration of the high-concentration reagent. The reagent evaluating unit 246 calculates the actual concentration of the high-concentration reagent based on the difference value (Mh-M0).

Then, the reagent evaluating unit 246 determines whether the concentration of the low-concentration reagent calculated in Step S226 is within a predetermined allowable range (Step S228). The allowable range is the concentration range of the low-concentration reagent in which an error can be accurately specified in the error specifying process. In the case where the amount of light emitted from the antigen 62a is proportional to the concentration thereof and the amount of light emitted during the manufacture of the low-concentration reagent is 100,000 counts, when the amount of light actually emitted from the reagent varies within $\pm 10\%$ of the original amount of light emitted, that is, in the range of 90,000 to 110,000 counts, it is possible to perform the error specifying process. Therefore, when a value obtained by subtracting the measurement result of the zero-concentration reagent measuring process from the measurement result of the low-concentration reagent measuring process is in the range of 90,000 to 110,000 counts, the reagent evaluating unit 246 determines that the low-concentration reagent can be used.

If the concentration of the low-concentration reagent is within the allowable range (Step S228: Yes), the reagent evaluating unit 246 considers that the concentration of the low-concentration reagent is maintained at a value suitable to accurately specify an error in the error specifying process. Therefore, the reagent evaluating unit 246 evaluates that the low-concentration reagent is normal (Step S230). Then, the reagent evaluating unit 246 determines that the low-concentration reagent can be used (Step S232).

Conversely, if the concentration of the low-concentration reagent is not within the allowable range (Step S228: No), the reagent evaluating unit 246 considers that the concentration of the low-concentration reagent is not maintained at a value suitable to accurately specify an error in the error specifying process. Therefore, the reagent evaluating unit 246 evaluates that the low-concentration reagent is defective (Step S234). As a result, the reagent evaluating unit 246 determines that the low-concentration reagent cannot be used (Step S236).

Then, the reagent evaluating unit 246 determines whether the concentration of the high-concentration reagent calculated in Step S226 is within a predetermined allowable range (Step S238). The allowable range is the concentration range of the high-concentration reagent in which an error can be accurately specified in the error specifying process. In the case where the amount of light emitted from the antigen 62b is proportional to the concentration thereof and the amount of light emitted during the manufacture of the high-concentration reagent is 1,000,000 counts, when the amount of light actually emitted from the reagent varies within $\pm 10\%$ of the original amount of light emitted, that is, in the range of 900,000 to 1,100,000 counts, it is possible to perform the error specifying process. Therefore, when a value obtained by subtracting the measurement result of the zero-concentration reagent measuring process from the measurement result of the high-concentration reagent measuring process is in the range of 900,000 to 1,100,000 counts, the reagent evaluating unit 246 determines that the high-concentration reagent can be used.

If the concentration of the high-concentration reagent is within the allowable range (Step S238: Yes), the reagent evaluating unit 246 considers that the concentration of the high-concentration reagent is maintained at a value suitable to accurately specify an error in the error specifying process. Therefore, the reagent evaluating unit 246 evaluates that the high-concentration reagent is normal (Step S240). As a result, the reagent evaluating unit 246 determines that the high-concentration reagent can be used (Step S242).

Conversely, if the concentration of the high-concentration reagent is not within the allowable range (Step S238: No), the reagent evaluating unit 246 considers that the concentration of the high-concentration reagent is not maintained at a value suitable to accurately specify an error in the error specifying process. Therefore, the reagent evaluating unit 246 evaluates that the high-concentration reagent is defective (Step S244). As a result, the reagent evaluating unit 246 determines that the high-concentration reagent cannot be used (Step S246). When receiving information indicating that the reagent cannot be used from the reagent evaluating unit 246, the process control unit 42 and the specifying unit 245 do not use the low-concentration reagent and the high-concentration reagent that have been determined to be unavailable.

As such, the analyzer 201 according to the second embodiment measures the amount of light emitted from the zero-concentration reagent to calculate the concentration of each of the low-concentration reagent and the high-concentration reagent, and individually evaluates the low-concentration reagent and the high-concentration reagent. Therefore, according to the analyzer 201, it is possible to continuously perform the error specifying process by replacing only the reagent that is determined to be unavailable. Therefore, it is possible to reduce the cost of replacing the reagent, as compared to the first embodiment in which it is necessary to replace both the low-concentration reagent and the high-concentration reagent.

(Third embodiment)

Next, a third embodiment will be described. In the third embodiment, the measurement result in a measurement process for error specification is corrected based on the concentration of each reagent or the concentration ratio calculated in a reagent evaluating process. In this way, it is possible to more accurately specify an error.

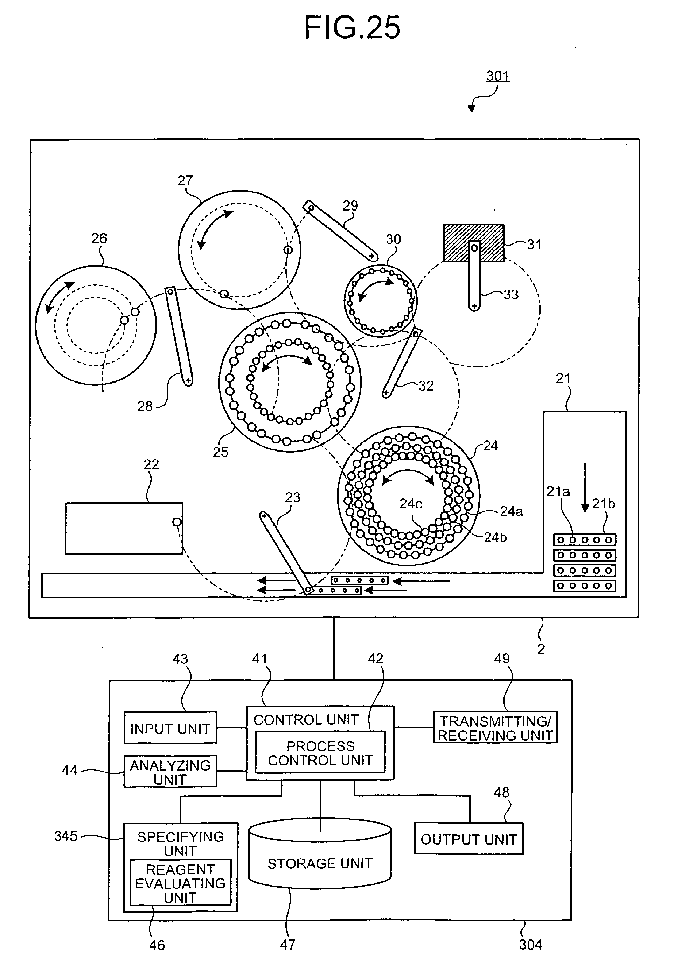

FIG. 25 is a diagram schematically illustrating the structure of an analyzer according to the third embodiment. As shown in FIG. 25, an analyzer 301 according to the third embodiment includes a specifying unit 345, instead of the specifying unit 45 of the control system 4 shown in FIG. 1. The specifying unit 345 corrects the measurement result in the measurement process for acquiring a BF reference or the measurement process for acquiring a probe reference, based on the concentration ratio of a low-concentration reagent and a high-concentration reagent calculated by the reagent evaluating unit 46. Then, the specifying unit 345 uses the corrected value as the reference value of the BF cleaning process or the reference value of the probe cleaning process to specify the error of the BF cleaning process or the probe cleaning process.

Next, the procedure of an error specifying process of the analyzer 301 will be described with reference to FIG.

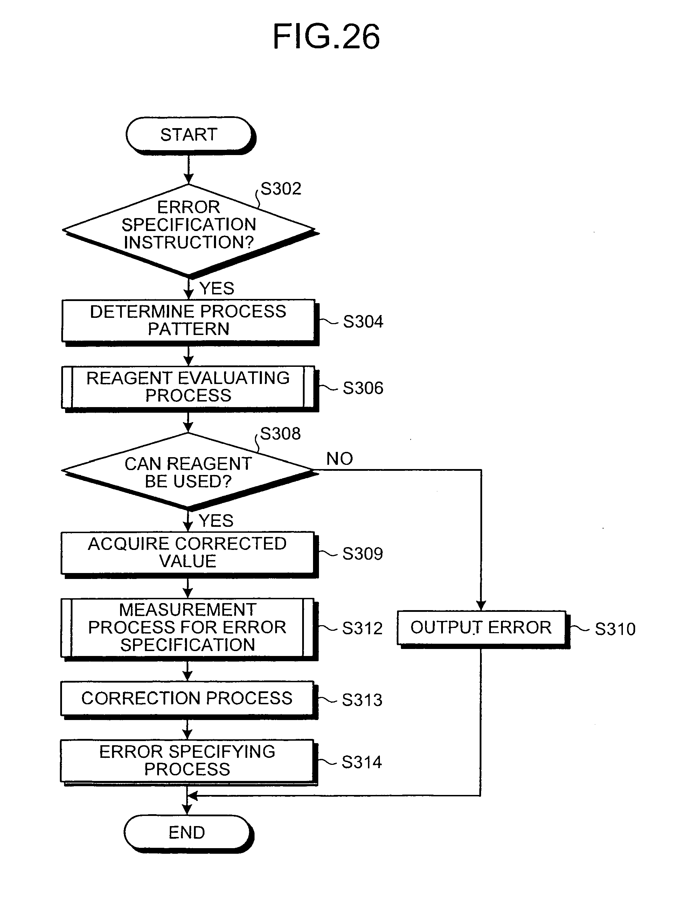

26. As shown in FIG. 26, the analyzer 301 performs the same procedure as that in Steps S2 to S6 shown in FIG. 2 to perform a process of determining whether an instruction to specify an error is input (Step S302), a process pattern determining process (Step S304), and a reagent evaluating process by the reagent evaluating unit 46 (Step S306). Then, the specifying unit 345 corrects the concentration ratio of the low-concentration reagent and the high-concentration reagent calculated in the reagent evaluating unit 46 to obtain a corrected value.

Then, similar to Step S8 shown in FIG. 2, the specifying unit 345 determines whether the low-concentration reagent and the high-concentration reagent can be used based on the evaluation result of the reagent evaluating unit 46 (Step S308). If the specifying unit 345 determines that the low-concentration reagent and the high-concentration reagent cannot be used (Step S308: No), similar to Step S10 shown in FIG. 2, the output unit 48 performs an error output process (Step S310).

Conversely, if it is determined that the low-concentration reagent and the high-concentration reagent can be used (Step S308: Yes), the specifying unit 345 acquires the concentration ratio of the low-concentration reagent and the high-concentration reagent calculated by the reagent evaluating unit 46 as a correction value for the measured value in the measurement process for error specification (Step S309). Then, each component of the measurement system 2 performs the same procedure as that in Step S12 shown in FIG. 2 using the low-concentration reagent and the high-concentration reagent that have been evaluated to be available by the reagent evaluating unit 46, under the control of the process control unit 42, thereby performing the measurement process for error specification (Step S312).

Then, the specifying unit 345 performs a correction process of correcting the reference value obtained in the measurement process for error specification (Step S313). The correction process corrects the reference value obtained in the measurement process for error specification based on the concentration ratio calculated by the reagent evaluating unit 46 in the reagent evaluating process.

Next, an example is described in which, when the amounts of light emitted from the antigens 62a and 62b are proportional to the concentrations of the antigens 62a and 62b and the ratio of the amount of light emitted from the low-concentration reagent and the amount of light emitted from the high-concentration reagent is originally set to 1:100,000, the ratio of the amounts of light calculated in the reagent evaluating process (Step S306) is 1:90,000. In this case, it is considered that the amount of light emitted from the high-concentration reagent is 10% less than the original amount of light emitted from the high-concentration reagent due to a variation in the active state of the high-concentration reagent. In this case, the specifying unit 345 reduces the measured value of the low-concentration reagent obtained in the measurement process for acquiring a BF reference or the measurement process for acquiring a probe reference in the measurement process for error specification (Step S312) by 10%, according to the decreasing rate of the amount of light emitted from the high-concentration reagent. The specifying unit 345 uses the corrected value as the reference value.

When the ratio of the amount of light emitted from the low-concentration reagent and the amount of light emitted from the high-concentration reagent calculated in the reagent evaluating process (Step S306) is 1:110,000, it is considered that the amount of light emitted from the low-concentration reagent is 10% less than the original amount of light emitted from the low-concentration reagent due to a variation in the active state of the low-concentration reagent. In this case, the specifying unit 345 increases the measured value of the low-concentration reagent obtained in the measurement process for acquiring a BF reference or the measurement process for acquiring a probe reference in the measurement process for error specification (Step S312) by 10%, according to the decreasing rate of the amount of light emitted from the low-concentration reagent. The specifying unit 345 uses the corrected value as the reference value.

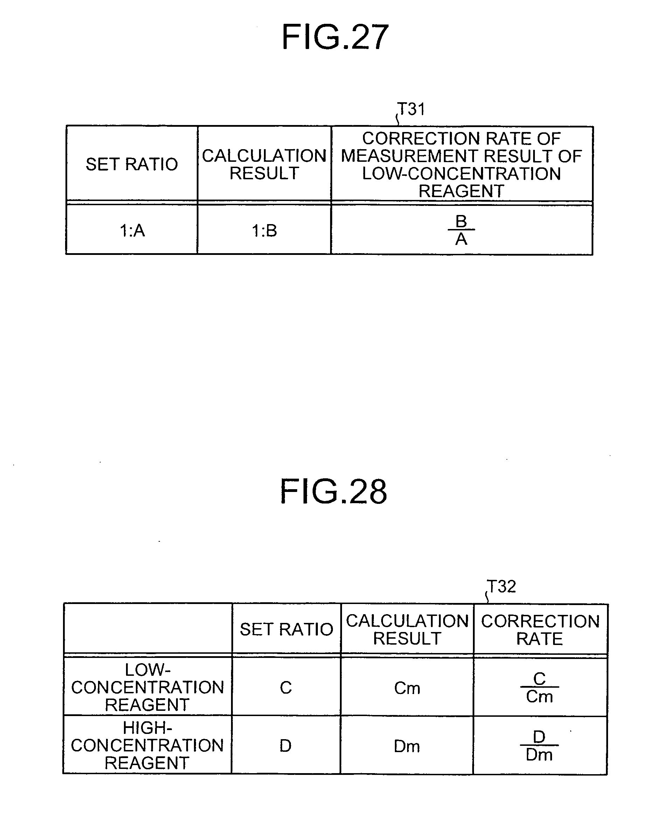

That is, as shown in a table T31 of FIG. 27, if the original concentration ratio of the low-concentration reagent and the high-concentration reagent is set to 1:A when the active states of the low-concentration reagent and the high-concentration reagent do not vary and the concentration ratio of the low-concentration reagent and the high-concentration reagent calculated from the measurement result in the reagent evaluating process (Step S306) is 1:B, the correction rate of the measurement result of the low-concentration reagent obtained in the measurement process for acquiring a BF reference or the measurement process for acquiring a probe reference in the measurement process for error specification (Step S312) is B/A.

The specifying unit 345 performs the same procedure as that in Step S14 shown in FIG. 2 using the corrected value as the reference value, thereby performing the error specifying process (Step S314). The specifying unit 345 uses the reference value that is corrected based on the concentration ratio calculated in the reagent evaluating process. Therefore, it is possible to reduce an error in error specification due to the error of the concentration ratio.

As such, the analyzer 301 according to the third embodiment corrects the reference value of the measurement process for error specification with simple calculation and then performs the error specifying process. Therefore, it is possible to more accurately perform an error specifying process.

In the third embodiment, the analyzer 301 corrects the reference value based on the concentration ratio calculated by the reagent evaluating unit 46. However, the specifying unit 345 may include the reagent evaluating unit 246 instead of the reagent evaluating unit 46 and correct the measurement result in the measurement process for error specification based on the actual concentration of each reagent calculated by the reagent evaluating unit 246.

In this case, the specifying unit 345 corrects the measurement result of the low-concentration reagent obtained in the measurement process for acquiring a BF reference or the measurement process for acquiring a probe reference in the measurement process for error specification (Step S312), based on the actual concentration of the low-concentration reagent calculated by the reagent evaluating unit 246. Specifically, as shown in a table T32 of FIG. 28, when a set value, which is the concentration value of the low-concentration reagent during manufacture, is C and calculation result of the actual concentration of the low-concentration reagent calculated in the reagent evaluating process is Cm, the correction rate of the measured value of the low-concentration reagent obtained in the measurement process for acquiring a BF reference or the measurement process for acquiring a probe reference is C/Cm. The specifying unit 345 multiplies the measured value of the low-concentration reagent obtained in the measurement process for acquiring a BF reference or the measurement process for acquiring a probe reference by C/Cm to obtain a corrected value, and uses the corrected value as the reference value. For example, when the amount of light emitted from the low-concentration reagent during manufacture is 100,000 counts and the value of the low-concentration reagent actually measured in the reagent evaluating process is 90,000 counts, a value obtained by multiplying the actually measured value by 10/9 is used as the reference value.

Furthermore, the specifying unit 345 corrects the measured result of the measured value for error specification obtained in the measurement process for specifying a BF error or the measurement process for specifying a probe error in the error specifying process (Step S312), based on the actual concentration of the high-concentration reagent calculated by the reagent evaluating unit 246. Specifically, as shown in the table T32 of FIG. 28, when a set value, which is the concentration value of the high-concentration reagent during manufacture, is D and the calculation result of the actual concentration of the high-concentration reagent calculated in the reagent evaluating process is Dm, the correction rate of the measured value for error specification obtained in the measurement process for specifying a BF error or the measurement process for specifying a probe error is D/Dm. The specifying unit 345 multiplies the measured value of the low-concentration reagent obtained in the measurement process for specifying a BF error or the measurement process for specifying a probe error by D/Dm to obtain a corrected value, and uses the corrected value as the measured value for error specification.

As such, when the actual concentrations of the low-concentration reagent and the high-concentration reagent calculated by the reagent evaluating unit 246 are used, the reference value and the measured value for error specification are corrected, and then error specification is performed. Therefore, it is possible to more accurately specify an error.

In the first to third embodiments, as shown in FIGS. 2, 22, and 26, after the reagent evaluating process is performed, the measurement process for error specification is performed using the reagent that has been determined to be normal. However, the invention is not limited thereto. For example, after the measurement process for error specification is performed, the performances of the low-concentration reagent and the high-concentration reagent used in the measurement process for error specification may be evaluated.

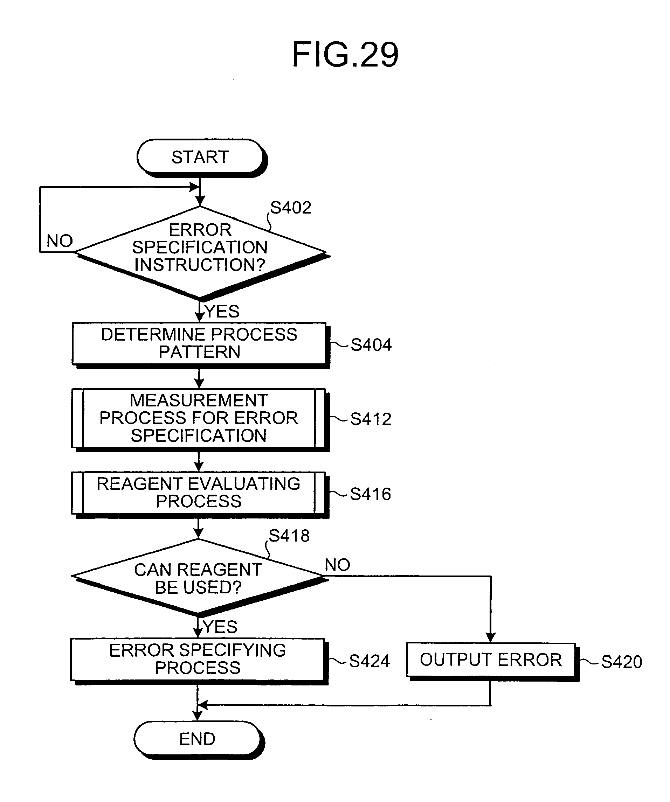

For example, as shown in FIG. 29, the analyzer 1 performs the same procedure as that in Steps S2, S4, and S12 shown in FIG. 2 to perform a process of determining whether an instruction to specify an error is input (Step S402), a process pattern determining process (Step S404) and a measurement process for error specification (Step S412). Then, the analyzer 1 performs the same procedure as that in Step S6 shown in FIG. 2 to perform a reagent evaluating process (Step S406). Then, the specifying unit 45 determines whether the low-concentration reagent and the high-concentration reagent can be used based on the evaluation result of the reagent evaluating unit 46 (Step S418).

If the specifying unit 45 determines that the low-concentration reagent and the high-concentration reagent cannot be used (Step S418: No), the output unit 48 performs the same procedure as that in Step S10 shown in FIG. 2 to perform an error output process (Step S420). Conversely, if the reagent evaluating unit 46 determines that both the low-concentration reagent and the high-concentration reagent can be used (Step S418: Yes), the specifying unit 45 performs the same procedure as that in Step S14 shown in FIG. 2 to perform an error specifying process (Step S424). In this case, the specifying unit 45 performs the error specifying process after it is determined that both the low-concentration reagent and the high-concentration reagent can be used. Therefore, it is possible to accurately specify an error. The analyzer 1 may perform the reagent evaluating process after the error specifying process, not before the error specifying process, to verify the error specification result in the error specifying process ex post facto.

The analyzers 1, 201, and 301 according to the above-described embodiments may be implemented by executing a program prepared in advance with a computer system, such as a personal computer or a workstation. The computer system reads the program recorded on a predetermined recording medium and executes the program to implement the operation of the analyzer. Examples of the predetermined recording medium include 'portable physical media', such as a flexible disk (FD), a CD-ROM, an MO disk, a DVD disk, a magneto-optical disk, and an IC card, and recording media on which computer readable programs are recorded, such as a hard disk drive (HDD) that is provided in the computer system and a 'communication medium' that temporarily stores programs when the programs are transmitted. The computer system acquires programs from another computer system that is connected thereto through a network and executes the acquired programs to implement the operation of the analyzer.

INDUSTRIAL APPLICABILITY

As such, the analyzer according to embodiments of the invention is useful for a medical analyzer that does not include a colorimeter. Particularly, the analyzer is applicable to easily and rapidly acquire the error of the analyzer.

**Claims**

1. An error specifying method of specifying an error of an analyzer that analyzes a specimen based on an optical measurement, the method specifying an error of an analysis process related to removal of a high-concentration reagent based on a reference value, which is a measurement result obtained by use of a low-concentration reagent containing a predetermined low-concentration composition, and a measured value for error specification, which is a measurement result obtained by an analysis process using the high-concentration reagent containing a predetermined high-concentration composition, the method comprising:

   a low-concentration reagent measuring step of performing an analysis process, which causes the composition to emit light, on the low-concentration reagent and measuring an amount of light emitted in order to acquire performance of the low-concentration reagent;
   a high-concentration reagent measuring step of performing the analysis process, which causes the composition to emit light, on the high-concentration reagent and measuring an amount of light emitted in order to acquire performance of the high-concentration reagent; and
   a reagent evaluating step of evaluating the performance of the low-concentration reagent and the performance of the high-concentration reagent based on the measurement result in the low-concentration reagent measuring step and the measurement result in the high-concentration reagent measuring step.

2. The error specifying method according to claim 1, further comprising
   a measuring step for acquiring a reference value of acquiring the measurement result obtained by the use of the low-concentration reagent as the reference value;
   a measuring step for error specification of acquiring the measurement result obtained by the analysis process using the high-concentration reagent as the measured value for error specification; and
   an error specifying step of specifying the error of the analysis process related to the removal of the high-concentration reagent based on whether the measured value for error specification is within an allowable range of the reference value,
   wherein the low-concentration reagent measuring step, the high-concentration reagent measuring step, and the reagent evaluating step are performed before or after the measuring step for acquiring a reference value and the measuring step for error specification.

3. The error specifying method according to claim 1 or 2,
   wherein the reagent evaluating step calculates a concentration ratio of the compositions in the low-concentration reagent and the high-concentration reagent based on the measurement result in the low-concentration reagent measuring step and the measurement result in the high-concentration reagent measuring step, and
   when the calculated concentration ratio is not within a predetermined allowable range, it is evaluated that at least one of the low-concentration reagent and the high-concentration reagent is defective.

4. The error specifying method according to claim 1 or 2, further comprising
   a zero-concentration reagent measuring step of performing the analysis process, which causes the composition to emit light, on a zero-concentration reagent that does not contain the composition and measuring an amount of light emitted,
   wherein the reagent evaluating step comprises
   a low-concentration reagent evaluating step of calculating the concentration of the composition in the low-concentration reagent based on a value obtained by subtracting a measurement result in the zero-concentration reagent measuring step from the measurement result in the low-concentration reagent measuring step, and evaluating that the low-concentration reagent is defective when the calculated concentration is not within a first predetermined allowable range; and
   a high-concentration reagent evaluating step of calculating the concentration of the composition in the high-concentration reagent based on a value obtained by subtracting the measurement result in the zero-concentration reagent measuring step from the measurement result in the high-concentration reagent measuring step, and evaluating that the high-concentration reagent is defective when the calculated concentration is not within a second predetermined allowable range.

5. The error specifying method according to claim 3, further comprising
a correction step of correcting the measurement result in the measuring step for acquiring a reference value based on the concentration ratio calculated in the reagent evaluating step,
wherein the error specifying step specifies the error of the analysis process related to the removal of the high-concentration reagent using the corrected value corrected in the correction step as the reference value.

6. The error specifying method according to claim 4, further comprising
a first correction step of correcting the measurement result in the measuring step for acquiring a reference value based on the concentration of the composition in the low-concentration reagent, which is calculated in the low-concentration reagent evaluating step; and
a second correction step of correcting the measurement result in the measuring step for error specification based on the concentration of the composition in the high-concentration reagent, which is calculated in the high-concentration reagent evaluating step,
wherein the error specifying step uses the corrected value corrected in the first correction step as the reference value, and uses the corrected value corrected in the second correction step as the measured value for error specification to specify the error of the analysis process related to the removal of the high-concentration reagent.

7. The error specifying method according to any one of claims 1 to 6, further comprising
a dilution rate setting step of setting a dilution rate of the high-concentration reagent based on the measured value of light emitted during manufacture of the high-concentration reagent and a range in which a photometric system of the analyzer can measure light,
wherein the high-concentration reagent measuring step performs the analysis process of causing the composition to emit light on the high-concentration reagent which is diluted at the dilution rate set in the dilution rate setting step, and measures the amount of light emitted.

8. The error specifying method according to any one of claims 2 to 7,
wherein the measuring step for acquiring a reference value comprises
a first measuring step of measuring the amount of light emitted by the analysis process, which causes the composition in the low-concentration reagent to emit light, as the reference value,
the measuring step for error specification comprises
a removal step of performing the same process as a removal process of removing an unreacted material in a reaction vessel included in analysis processes for the specimen to remove the composition in the high-concentration reagent accommodated in the reaction vessel, after the high-concentration reagent is injected into the reaction vessel; and
a second measuring step of measuring the amount of light emitted by the analysis process that causes the composition to emit light as the measured value for error specification, and
in the error specifying step, when the measured value for error specification is not within the allowable range, it is specified that there is a removal error in the removal process included in the analysis processes for the specimen.

9. The error specifying method according to claim 8,
wherein the error specifying step calculates a carry-over value of the removal process based on a value obtained by dividing the measured value for error specification by the measurement result obtained in the high-concentration reagent measuring step.

10. The error specifying method according to claim 8 or 9,
wherein a first removal process and a second removal process are performed as the removal process, and
the removal step is any one of a first removal step in which the same process as the first removal process is performed, a second removal step in which the same process as the second removal process is performed, and a third removal step in which the same processes as the first removal process and the second removal process are performed.

11. The error specifying method according to claim 10,
wherein, in the measuring step for error specification, when the removal step is the first removal step and the measured value for error specification is not within the allowable range, it is specified that there is a removal error in the first removal process of the analysis process for the specimen,
when the removal step is the second removal step and the measured value for error specification is not within the allowable range, it is specified that there is a removal error in the second removal process of the analysis process for the specimen, and

when the removal step is the third removal step and the measured value for error specification is not within the allowable range, it is specified that there is a removal error in the first removal process and/or the second removal process of the analysis process for the specimen.

12. The error specifying method according to any one of claims 2 to 7,

wherein the measuring step for acquiring a reference value comprises

a first measuring step of measuring the amount of light emitted by the analysis process, which causes the composition in the low-concentration reagent to emit light, as the reference value,

the measuring step for error specification comprises

a high-concentration reagent injection step of injecting the high-concentration reagent into a reaction vessel using a liquid injection system that injects a liquid into the reaction vessel;

a zero-concentration reagent injection step of injecting a zero-concentration reagent that does not contain the composition into a reaction vessel different from the reaction vessel having the high-concentration reagent injected thereinto, using the liquid injection system which is cleaned after the high-concentration reagent injection step; and

a second measuring step of measuring, as the measured value for error specification, the amount of light emitted from the reaction vessel having the zero-concentration reagent injected thereinto after the analysis process of causing the composition to emit light is performed, and

in the error specifying step, when the measured value for error specification is not within the allowable range, it is specified that there is an error in a cleaning process of the liquid injection system in the analysis process for the specimen.

13. The error specifying method according to claim 12,

wherein the error specifying step calculates a carry-over value of the removal process based on a value obtained by dividing the measured value for error specification by the measurement result obtained in the high-concentration reagent measuring step.

14. An analyzer for analyzing a specimen based on an optical measurement, the analyzer specifying an error of an analysis process related to removal of a high-concentration reagent based on a reference value, which is a measurement result obtained by use of a low-concentration reagent containing a predetermined low-concentration composition, and a measured value for error specification, which is a measurement result obtained by an analysis process using the high-concentration reagent containing a predetermined high-concentration composition, the analyzer comprising:

a measuring unit that performs a low-concentration reagent measuring process of performing an analysis process, which causes the composition to emit light, on the low-concentration reagent and measuring an amount of light emitted in order to acquire performance of the low-concentration reagent and a high-concentration reagent measuring process of performing the analysis process, which causes the composition to emit light, on the high-concentration reagent and measuring an amount of light emitted in order to acquire performance of the high-concentration reagent; and

a reagent evaluating unit that evaluates the performance of the low-concentration reagent and the performance of the high-concentration reagent based on the measurement result of the low-concentration reagent measuring process and the measurement result of the high-concentration reagent measuring process obtained by the measuring unit.

15. The analyzer according to claim 14, further comprising

a specifying unit that specifies the error of the analysis process related to the removal of the high-concentration reagent,

wherein the measuring unit performs a measurement process for acquiring a reference value that acquires the measurement result obtained by the use of the low-concentration reagent as the reference value and a measurement process for error specification that acquires the measurement result obtained by the analysis process using the high-concentration reagent as the measured value for error specification, and

the measuring unit performs the low-concentration reagent measuring process and the high-concentration reagent measuring process before or after the measurement process for acquiring a reference value and the measurement process for error specification,

the reagent evaluating unit evaluates the performance of the low-concentration reagent and the performance of the high-concentration reagent before or after the measurement process for acquiring a reference value and the measurement process for error specification, and

the specifying unit specifies the error of the analysis process related to the removal of the high-concentration reagent based on whether the measured value for error specification obtained by the measuring unit is within an allowable range of the reference value.

16. The analyzer according to claim 14 or 15,
wherein the reagent evaluating unit calculates a concentration ratio of the compositions in the low-concentration reagent and the high-concentration reagent based on the measurement result of the low-concentration reagent measuring process and the measurement result of the high-concentration reagent measuring process obtained by the measuring unit, and
when the calculated concentration ratio is not within a predetermined allowable range, the reagent evaluating unit evaluates that at least one of the low-concentration reagent and the high-concentration reagent is defective.

17. The analyzer according to claim 14 or 15,
wherein the measuring unit performs a zero-concentration reagent measuring process, in which the analysis process of causing the composition to emit light is performed on a zero-concentration reagent that does not contain the composition and the amount of light emitted is measured, and
the reagent evaluating unit performs a low-concentration reagent evaluating process that calculates the concentration of the composition in the low-concentration reagent based on a value obtained by subtracting a measurement result of the zero-concentration reagent measuring process from the measurement result of the low-concentration reagent measuring process obtained by the measuring unit and evaluates that the low-concentration reagent is defective when the calculated concentration is not within a first predetermined allowable range, and a high-concentration reagent evaluating process that calculates the concentration of the composition in the high-concentration reagent based on a value obtained by subtracting the measurement result of the zero-concentration reagent measuring step from the measurement result of the high-concentration reagent measuring step and evaluates that the high-concentration reagent is defective when the calculated concentration is not within a second predetermined allowable range.

18. The analyzer according to claim 16,
wherein the specifying unit corrects the measurement result of the measurement process for acquiring a reference value obtained by the measuring unit based on the concentration ratio calculated by the reagent evaluating unit, and specifies the error of the analysis process related to the removal of the high-concentration reagent using the corrected value as the reference value.

19. The analyzer according to claim 17,
wherein the specifying unit performs a first correction process that corrects the measurement result of the measurement process for acquiring a reference value obtained by the measuring unit based on the concentration of the composition in the low-concentration reagent which is calculated by the reagent evaluating unit, and a second correction process that corrects the measurement result of the measurement process for error specification obtained by the measuring unit based on the concentration of the composition in the high-concentration reagent which is calculated by the reagent evaluating unit, and
the specifying unit uses the corrected value corrected in the first correction process as the reference value, and uses the corrected value corrected in the second correction process as the measured value for error specification to specify the error of the analysis process related to the removal of the high-concentration reagent.

20. The analyzer according to any one of claims 14 to 19,
wherein the reagent evaluating unit sets a dilution rate of the high-concentration reagent based on the measured value of light emitted during manufacture of the high-concentration reagent and a range in which a photometric system of the analyzer can measure light, and
the measuring unit performs the analysis process that causes the composition to emit light on the high-concentration reagent which is diluted at the dilution rate set by the reagent evaluating unit, and measures the amount of light emitted.

21. The analyzer according to any one of claims 15 to 20, further comprising
a removal unit that performs a removal process of removing an unreacted material in a reaction vessel, the removal process included in analysis processes for the specimen,
wherein the removal unit performs the same process as the removal process to remove the composition in the high-concentration reagent accommodated in the reaction vessel after the high-concentration reagent is injected into the reaction vessel,
the measuring unit measures the amount of light emitted by the analysis process that causes the composition in the low-concentration reagent to emit light as the reference value and measures, as the measured value for error specification, the amount of light emitted by the analysis process that causes the composition to emit light after the composition in the high-concentration reagent is removed by the removal unit, and

when the measured value for error specification is not within the allowable range, the specifying unit specifies that there is a removal error in the removal process included in the analysis processes for the specimen.

22. The analyzer according to claim 21,
wherein the specifying unit calculates a carry-over value of the removal process based on a value obtained by dividing the measured value for error specification by the measurement result obtained in the high-concentration reagent measuring process.

23. The analyzer according to claim 21 or 22,
wherein a first removal process and a second removal process are performed as the removal process, and the removal unit performs the same process as the first removal process, the same process as the second removal process, or the same processes as the first removal process and the second removal process to remove the composition in the high-concentration reagent in the reaction vessel.

24. The analyzer according to claim 23,
wherein, when the removal unit performs the same process as the first removal process and the measured value for error specification is not within the allowable range, the specifying unit specifies that there is a removal error in the first removal process of the analysis process for the specimen,
when the removal unit performs the same process as the second removal process and the measured value for error specification is not within the allowable range, the specifying unit specifies that there is a removal error in the second removal process of the analysis process for the specimen, and
when the removal unit performs the same processes as the first removal process and the second removal process and the measured value for error specification is not within the allowable range, the specifying unit specifies that there is a removal error in the first removal process and/or the second removal process of the analysis process for the specimen.

25. The analyzer according to any one of claims 15 to 20, further comprising
a liquid injection unit that injects a liquid into a reaction vessel,
wherein the liquid injection unit injects the high-concentration reagent into the reaction vessel, is cleaned, and injects a zero-concentration reagent that does not contain the composition into a reaction vessel different from the reaction vessel having the high-concentration reagent injected thereinto,
the measuring unit measures the amount of light emitted by the analysis process that causes the composition in the low-concentration reagent to emit light as the reference value, and measures, as the measured value for error specification, the amount of light emitted from the reaction vessel having the zero-concentration reagent injected thereinto after the analysis process of causing the composition to emit light is performed, and
when the measured value for error specification is not within the allowable range, the specifying unit specifies that there is an error in a cleaning process of the liquid injection unit in the analysis process for the specimen.

26. The analyzer according to claim 25,
wherein the specifying unit calculates a carry-over value of the removal process based on a value obtained by dividing the measured value for error specification by the measurement result obtained in the high-concentration reagent measuring process.

# FIG.1

# FIG.2

```
                    ┌──────────────┐
                    │    START     │
                    └──────┬───────┘
                           │
              ┌────────────▼────────────┐  S2
      NO      │        ERROR            │
    ┌─────────┤    SPECIFICATION        │
    │         │     INSTRUCTION?        │
    │         └────────────┬────────────┘
    │                      │YES
    │         ┌────────────▼────────────┐
    │         │  DETERMINE PROCESS      │  S4
    │         │       PATTERN           │
    │         └────────────┬────────────┘
    │         ┌────────────▼────────────┐
    │         │  REAGENT EVALUATING     │  S6
    │         │       PROCESS           │
    │         └────────────┬────────────┘
    │                      │
    │         ┌────────────▼────────────┐  S8       NO
    │         │     CAN REAGENT         ├──────────────┐
    │         │      BE USED?           │              │
    │         └────────────┬────────────┘              │
    │                      │YES                        │
    │         ┌────────────▼────────────┐    ┌─────────▼─────────┐
    │         │    MEASUREMENT          │    │   OUTPUT ERROR    │ S10
    │         │  PROCESS FOR ERROR      │S12 └─────────┬─────────┘
    │         │   SPECIFICATION         │              │
    │         └────────────┬────────────┘              │
    │         ┌────────────▼────────────┐              │
    │         │  ERROR SPECIFYING       │  S14         │
    │         │       PROCESS           │              │
    │         └────────────┬────────────┘              │
    │                      ◄─────────────────────────── ┘
    │              ┌───────▼───────┐
    │              │     END       │
    │              └───────────────┘
```

31

# FIG.3

```
        ┌─────────────────┐
        │     REAGENT     │
        │   EVALUATING    │
        │     PROCESS     │
        └─────────────────┘
                 │
                 ▼
     ┌──────────────────────┐
     │  LOW-CONCENTRATION   │
     │  REAGENT MEASURING   │  ~ S22
     │       PROCESS        │
     └──────────────────────┘
                 │
                 ▼
     ┌──────────────────────┐
     │  HIGH-CONCENTRATION  │
     │  REAGENT MEASURING   │  ~ S24
     │       PROCESS        │
     └──────────────────────┘
                 │
                 ▼
     ┌──────────────────────┐
     │      CALCULATE       │
     │ CONCENTRATION RATIO  │  ~ S26
     └──────────────────────┘
                 │
                 ▼
```

S28

IS CONCENTRATION RATIO WITHIN ALLOWABLE RANGE?

NO

YES

| IT IS EVALUATED THAT REAGENT IS NORMAL | ~ S30 | IT IS EVALUATED THAT REAGENT IS DEFECTIVE | ~ S34 |

| REAGENT CAN BE USED | ~ S32 | REAGENT CANNOT BE USED | ~ S36 |

RETURN

FIG.4

(1)   (2)   (3)   (4)   (5)   (6)   (7)

EP 2 175 259 A1

# FIG.5

(1)　　　(2)　　　(3)　　　(4)

# FIG.6

(1)　　　(2)　　　(3)　　　(4)

# FIG.7

MEASUREMENT
PROCESS FOR ERROR
SPECIFICATION

WHAT
IS ERROR
SPECIFICATION
TARGET? — S41

PROBE CLEANING
PROCESS

BF CLEANING
PROCESS

MEASUREMENT
PROCESS
FOR ACQUIRING
BF REFERENCE — S42

MEASUREMENT
PROCESS
FOR ACQUIRING
PROBE REFERENCE — S44

MEASUREMENT
PROCESS
FOR SPECIFYING
BF ERROR — S43

PROCESS USING
HIGH-
CONCENTRATION
REAGENT — S45

MEASUREMENT
PROCESS
FOR SPECIFYING
PROBE ERROR — S46

RETURN

# FIG.8

```
┌──────────────────────┐      ┌──────────────────────┐
│   MEASUREMENT        │      │   · MEASUREMENT      │
│   PROCESS FOR        │      │   PROCESS FOR        │
│   ACQUIRING          │      │ SPECIFYING BF ERROR  │
│   BF REFERENCE       │      │                      │
└──────────────────────┘      └──────────────────────┘
         │                              │  ⌐ S51
         │              ┌───────────────▼──────────────┐
         │              │   DISPENSE DUMMY REAGENT      │
         │              └───────────────┬──────────────┘
         │                              │  ⌐ S522
         │                   ┌──────────▼───────────┐
         │                   │   DISPENSE           │
         │                   │ HIGH-CONCENTRATION   │
         │                   │   REAGENT            │
         │                   └──────────┬───────────┘
         │                              │  ⌐ S53
┌────────▼──────────────────────────────▼──────────────┐
│     BF CLEANING PROCESS FOR SPECIFICATION             │
└────────┬─────────────────────────────┬───────────────┘
         │  ⌐ S541                      │  ⌐ S542
┌────────▼──────────┐        ┌──────────▼──────────────┐
│   DISPENSE        │        │  DISPENSE REAGENT       │
│ LOW-CONCENTRATION │        │  FOR SPECIFICATION      │
│   REAGENT         │        │                         │
└────────┬──────────┘        └──────────┬──────────────┘
         │                              │  ⌐ S55
┌────────▼──────────────────────────────▼──────────────┐
│             SECOND BF CLEANING                        │
└────────┬─────────────────────────────┬───────────────┘
         │                              │  ⌐ S56
┌────────▼──────────────────────────────▼──────────────┐
│             INJECT SUBSTRATE                          │
└────────┬─────────────────────────────┬───────────────┘
         │                              │  ⌐ S57
┌────────▼──────────────────────────────▼──────────────┐
│             MEASUREMENT                               │
└────────┬─────────────────────────────┬───────────────┘
         │                              │
┌────────▼──────────────────────────────▼──────────────┐
│             RETURN                                    │
└───────────────────────────────────────────────────────┘
```

# FIG.9

```
                    ┌─────────────────┐
                    │   BF CLEANING   │
                    │  PROCESS FOR    │
                    │ SPECIFICATION   │
                    └────────┬────────┘
                             │
                             ▼                    S531
      FIRST BF            ╱─────────╲        FIRST BF CLEANING AND
      CLEANING          ╱ SPECIFICATION ╲     SECOND BF CLEANING
      ◄───────────────◄    TARGET?       ►───────────────►
                          ╲             ╱
                           ╲───────────╱
                                │
                          SECOND BF
                          CLEANING
```

|  | |  |
|---|---|---|
| S532 | S533 | S534 |
| FIRST BF CLEANING | SECOND BF CLEANING | FIRST BF CLEANING |

```
                                              │
                                              ▼   S535
                                    ┌──────────────────┐
                                    │ SECOND BF CLEANING│
                                    └──────────────────┘
```

```
                    ┌─────────────────┐
                    │     RETURN      │
                    └─────────────────┘
```

# FIG.10

EP 2 175 259 A1

# FIG.11

EP 2 175 259 A1

# FIG.12

| SPECIFIC BF CLEANING PROCESS | MEASURED VALUE FOR ERROR SPECIFICATION | ERROR | |
|---|---|---|---|
| FIRST BF CLEANING | EQUAL TO OR MORE THAN 1.1 TIMES REFERENCE VALUE | FIRST BF CLEANING | R1 |
| SECOND BF CLEANING | EQUAL TO OR MORE THAN 1.1 TIMES REFERENCE VALUE | SECOND BF CLEANING | R2 |
| FIRST BF CLEANING AND SECOND BF CLEANING | EQUAL TO OR MORE THAN 1.2 TIMES REFERENCE VALUE | FIRST BF CLEANING AND SECOND BF CLEANING | R3 |

T1

# FIG.13

```
┌──────────────────┐   ┌──────────────────┐   ┌──────────────────┐
│   MEASUREMENT    │   │  PROCESS USING   │   │   MEASUREMENT    │
│     PROCESS      │   │HIGH-CONCENTRATION│   │     PROCESS      │
│  FOR ACQUIRING   │   │     REAGENT      │   │  FOR SPECIFYING  │
│ PROBE REFERENCE  │   │                  │   │   PROBE ERROR    │
└──────────────────┘   └──────────────────┘   └──────────────────┘
```

S61

**DISPENSE FIRST REAGENT**

| S621 | S622 | S623 |
|---|---|---|
| **DISPENSE LOW-CONCENTRATION REAGENT** | **DISPENSE HIGH-CONCENTRATION REAGENT** | **DISPENSE ZERO-CONCENTRATION REAGENT** |

S64

**DISPENSE SECOND REAGENT**

S65

**SECOND BF CLEANING**

S66

**INJECT SUBSTRATE**

| S671 | | S673 |
|---|---|---|
| **MEASUREMENT** | | **MEASUREMENT** |

**END**

# FIG.14

EP 2 175 259 A1

# FIG.15

# FIG.16

EP 2 175 259 A1

# FIG.17

# FIG.18

| MEASURED VALUE FOR ERROR SPECIFICATION | ERROR |
|---|---|
| EQUAL TO OR MORE THAN 1.1 TIMES REFERENCE VALUE | PROBE CLEANING ERROR |

# FIG.19

```
                    ┌──────────────┐
                    │    START     │
                    └──────────────┘
                            │
     ┌──────────────────────▼──────────
     │                              S102
     │            ╱ERROR╲
NO   │          ╱SPECIFICATION╲
◄────┤◄────────  INSTRUCTION?
     │          ╲            ╱
     │            ╲        ╱
     │                │YES
     │        ┌───────▼────────┐
     │        │DETERMINE PROCESS│ ~S104
     │        │    PATTERN      │
     │        └───────┬────────┘
     │        ┌───────▼────────┐
     │        │DILUTION RATE SETTING│ ~S105
     │        │    PROCESS      │
     │        └───────┬────────┘
     │        ┌───────▼────────┐
     │        │REAGENT EVALUATING│ ~S106
     │        │    PROCESS      │
     │        └───────┬────────┘
     │                │            S108
     │          ╱CAN REAGENT╲   NO
     │         ╱ BE USED?   ╲────────────┐
     │          ╲          ╱             │
     │                │YES               │
     │        ┌───────▼────────┐  ┌──────▼──────┐
     │        │MEASUREMENT     │  │OUTPUT ERROR │ ~S110
     │        │PROCESS FOR ERROR│~S112 └──────┬──────┘
     │        │SPECIFICATION   │         │
     │        └───────┬────────┘         │
     │        ┌───────▼────────┐         │
     │        │ERROR SPECIFYING│ ~S114   │
     │        │    PROCESS     │         │
     │        └───────┬────────┘         │
     │                │◄─────────────────┘
     │        ┌───────▼────────┐
              │     END        │
              └────────────────┘
```

# FIG.20

|  | LOWER LIMIT | UPPER LIMIT |
|---|---|---|
| MEASUREMENT RANGE | 1 | 500 |
| DILUTION RATE [TIMES] | 2 | 1,000,000 |

T3

Y1

| LOW-CONCENTRATION REAGENT | HIGH-CONCENTRATION REAGENT |
|---|---|
| 10 | 1,000,000 |

T4

[MEASURED VALUE]

Y2

| | LOW-CONCENTRATION REAGENT | HIGH-CONCENTRATION REAGENT |
|---|---|---|
| DILUTION RATE [TIMES] | - | 10,000 |

T5

# FIG.21

# FIG.22

START

S202
ERROR SPECIFICATION INSTRUCTION?

NO

YES

DETERMINE PROCESS PATTERN — S204

REAGENT EVALUATING PROCESS — S206

S208
CAN REAGENT BE USED?

NO

YES

MEASUREMENT PROCESS FOR ERROR SPECIFICATION — S212

OUTPUT ERROR — S210

ERROR SPECIFYING PROCESS — S214

END

# FIG.23

```
        ┌─────────────────┐
        │    REAGENT      │
        │  EVALUATING     │
        │   PROCESS       │
        └────────┬────────┘
                 ▼
    ┌────────────────────────┐
    │  ZERO-CONCENTRATION    │
    │  REAGENT MEASURING     │  ~S221
    │      PROCESS           │
    └───────────┬────────────┘
                ▼
    ┌────────────────────────┐
    │  LOW-CONCENTRATION     │
    │  REAGENT MEASURING     │  ~S222
    │      PROCESS           │
    └───────────┬────────────┘
                ▼
    ┌────────────────────────┐
    │  HIGH-CONCENTRATION    │
    │  REAGENT MEASURING     │  ~S224
    │      PROCESS           │
    └───────────┬────────────┘
                ▼
    ┌────────────────────────┐
    │  CALCULATE CONCENTRATION│  ~S226
    └───────────┬────────────┘
                ▼
```

S228

IS CONCENTRATION OF LOW-CONCENTRATION REAGENT WITHIN ALLOWABLE RANGE?

NO → IT IS EVALUATED THAT LOW-CONCENTRATION REAGENT IS DEFECTIVE ~S234 → LOW-CONCENTRATION REAGENT CANNNOT BE USED ~S236

YES → IT IS EVALUATED THAT LOW-CONCENTRATION REAGENT IS NORMAL ~S230 → LOW-CONCENTRATION REAGENT CAN BE USED ~S232

S238

IS CONCENTRATION OF HIGH-CONCENTRATION REAGENT WITHIN ALLOWABLE RANGE?

NO → IT IS EVALUATED THAT HIGH-CONCENTRATION REAGENT IS DEFECTIVE ~S244 → HIGH-CONCENTRATION REAGENT CANNNOT BE USED ~S246

YES → IT IS EVALUATED THAT HIGH-CONCENTRATION REAGENT IS NORMAL ~S240 → HIGH-CONCENTRATION REAGENT CAN BE USED ~S242

RETURN

# FIG.24

MEASURED VALUE

# FIG.25

# FIG.26

```
                        ┌─────────────┐
                        │    START    │
                        └─────────────┘
                               │
                               ▼
                        ╱──────────────╲  S302
                       ╱      ERROR      ╲
                      ╱   SPECIFICATION   ╲
                      ╲    INSTRUCTION?    ╱
                       ╲                  ╱
                        ╲────────────────╱
                               │ YES
                               ▼
                     ┌──────────────────┐
                     │ DETERMINE PROCESS│── S304
                     │     PATTERN      │
                     └──────────────────┘
                               │
                               ▼
                     ┌──────────────────┐
                     │ REAGENT EVALUATING│── S306
                     │     PROCESS      │
                     └──────────────────┘
                               │
                               ▼
                        ╱──────────────╲  S308
                       ╱  CAN REAGENT    ╲      NO
                       ╲    BE USED?      ╱─────────────┐
                        ╲────────────────╱              │
                               │ YES                    │
                               ▼                        │
                     ┌──────────────────┐               │
                     │ ACQUIRE CORRECTED│── S309         │
                     │      VALUE       │               │
                     └──────────────────┘               │
                               │                        ▼
                               ▼              ┌───────────────────┐
                     ┌──────────────────┐     │   OUTPUT ERROR    │── S310
                     │   MEASUREMENT    │     └───────────────────┘
                     │ PROCESS FOR ERROR│── S312         │
                     │  SPECIFICATION   │               │
                     └──────────────────┘               │
                               │                        │
                               ▼                        │
                     ┌──────────────────┐               │
                     │CORRECTION PROCESS│── S313         │
                     └──────────────────┘               │
                               │                        │
                               ▼                        │
                     ┌──────────────────┐               │
                     │ ERROR SPECIFYING │── S314         │
                     │     PROCESS      │               │
                     └──────────────────┘               │
                               │◄───────────────────────┘
                               ▼
                        ┌─────────────┐
                        │     END     │
                        └─────────────┘
```

# FIG.27

T31

| SET RATIO | CALCULATION RESULT | CORRECTION RATE OF MEASUREMENT RESULT OF LOW-CONCENTRATION REAGENT |
|-----------|--------------------|---------------------------------------------------------------------|
| 1:A | 1:B | $\dfrac{B}{A}$ |

# FIG.28

T32

| | SET RATIO | CALCULATION RESULT | CORRECTION RATE |
|---|-----------|--------------------|-----------------| 
| LOW-CONCENTRATION REAGENT | C | Cm | $\dfrac{C}{Cm}$ |
| HIGH-CONCENTRATION REAGENT | D | Dm | $\dfrac{D}{Dm}$ |

# FIG.29

## INTERNATIONAL SEARCH REPORT

| | International application No. |
| --- | --- |
| | PCT/JP2007/062312 |

A.  CLASSIFICATION OF SUBJECT MATTER
*G01N21/77*(2006.01)i, *G01N35/00*(2006.01)i

According to International Patent Classification (IPC) or to both national classification and IPC

B.  FIELDS SEARCHED

Minimum documentation searched (classification system followed by classification symbols)
G01N21/75-G01N21/83, G01N35/00

Documentation searched other than minimum documentation to the extent that such documents are included in the fields searched
Jitsuyo Shinan Koho           1922-1996   Jitsuyo Shinan Toroku Koho   1996-2007
Kokai Jitsuyo Shinan Koho     1971-2007   Toroku Jitsuyo Shinan Koho   1994-2007

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
| --- | --- | --- |
| A | JP 2003-57248 A  (Hitachi, Ltd.), 26 February, 2003 (26.02.03), Full text; all drawings | 1-26 |
| A | JP 9-210999 A  (Hitachi, Ltd.), 15 August, 1997 (15.08.97), Par. Nos. [0031] to [0035] | 1-26 |
| A | JP 2004-184141 A  (Hitachi High-technologies Corp.), 02 July, 2004 (02.07.04), Full text; all drawings | 1-26 |
| A | JP 3109443 U  (Hitachi High-technologies Corp.), 19 May, 2005 (19.05.05), Full text; all drawings | 1-26 |

| ☒ | Further documents are listed in the continuation of Box C. | ☒ | See patent family annex. |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search 05 July, 2007 (05.07.07) | Date of mailing of the international search report 17 July, 2007 (17.07.07) |
| --- | --- |
| Name and mailing address of the ISA/ Japanese Patent Office | Authorized officer |
| Facsimile No. | Telephone No. |

Form PCT/ISA/210 (second sheet) (April 2005)

**INTERNATIONAL SEARCH REPORT**

International application No.

PCT/JP2007/062312

C (Continuation).    DOCUMENTS CONSIDERED TO BE RELEVANT

| Category* | Citation of document, with indication, where appropriate, of the relevant passages | Relevant to claim No. |
|---|---|---|
| A | JP 6-331630 A  (Daikin Industries, Ltd.),<br>02 December, 1994 (02.12.94),<br>Full text; all drawings | 1-26 |

Form PCT/ISA/210 (continuation of second sheet) (April 2005)

| INTERNATIONAL SEARCH REPORT<br>Information on patent family members | International application No.<br>PCT/JP2007/062312 |
|---|---|

| | | | |
|---|---|---|---|
| JP 2003-57248 A | 2003.02.26 | (Family: none) | |
| JP 9-210999 A | 1997.08.15 | JP 3427606 B2 | 2003.07.22 |
| JP 2004-184141 A | 2004.07.02 | JP 3840450 B2 | 2006.11.01 |
| JP 3109443 U | 2005.05.19 | (Family: none) | |
| JP 6-331630 A | 1994.12.02 | (Family: none) | |

Form PCT/ISA/210 (patent family annex) (April 2005)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003083988 A